(19) Europäisches Patentamt
European Patent Office
Office européen des brevets

(11) Publication number : **0 641 797 A1**

# (12) EUROPEAN PATENT APPLICATION

(21) Application number : **94305665.5**

(51) Int. Cl.[6] : **C07D 501/46,** A61K 31/545

(22) Date of filing : **29.07.94**

(30) Priority : **30.07.93 JP 189902/93**

(43) Date of publication of application :
**08.03.95 Bulletin 95/10**

(84) Designated Contracting States :
**AT BE CH DE DK ES FR GB GR IE IT LI LU MC NL PT SE**

(71) Applicant : **KATAYAMA SEIYAKUSYO CO. Ltd.**
**202, 2-1-15, Himesato,**
**Nishiyodogawa-ku**
**Osaka-shi, Osaka-fu (JP)**

(71) Applicant : **Ajinomoto Co., Inc.**
**No. 15-1, Kyobashi 1-chome**
**Chuo-ku**
**Tokyo (JP)**

(72) Inventor : **Hayashi, Sadao, c/o Hirakata Kojo**
**Katayama Seiyakusyo Co. Ltd.,**
**1-12-3 Shodai-Tajika**
**Hirakata-shi, Osaka-fu (JP)**
Inventor : **Kurita, Yasuyuki, c/o Hirakata Kojo**
**Katayama Seiyakusyo Co. Ltd.,**
**1-12-3 Shodai-Tajika**
**Hirakata-shi, Osaka-fu (JP)**
Inventor : **Mizutani, Akihito, c/o Hirakata Kojo**
**Katayama Seiyakusho Co. Ltd.,**
**1-12-3 Shodai-Tajika**
**Hirakata-shi, Osaka-fu (JP)**
Inventor : **Nakanishi, Eiji, c/o Central Res. Lab.**
**Ajinomoto Co., Inc.,**
**No. 1-1 Suzuki-cho**
**Kawasaki-ku, Kawasaki-shi, Kanagawa-ken (JP)**
Inventor : **Okunishi, Masahiko, c/o Central Res. Lab.**
**Ajinomoto Co., Inc.,**
**No. 1-1 Suzuki-cho**
**Kawasaki-ku, Kawasaki-shi, Kanagawa-ken (JP)**

(74) Representative : **Nicholls, Kathryn Margaret et al**
**MEWBURN ELLIS**
**York House**
**23 Kingsway**
**London WC2B 6HP (GB)**

(54) **Cephem derivatives and antimicrobial agents containing the same.**

(57) Herein disclosed are novel cephem compounds represented by the following general formula (I), and antimicrobial agents which contains at least one of such novel cephem compounds as an active ingredient.

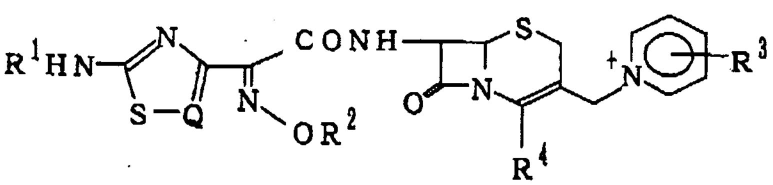

(I)

EP 0 641 797 A1

This invention relates to cephem compounds having antimicrobial activity, inter alia, antibacterial activity, and to antimicrobial agents which contain at least one of the compounds as an active ingredient.

Various cephem compounds or derivatives thereof have been hitherto synthesized which have a quaternary ammonium methyl group at the 3-position and, at the same time, a 2-(2-aminothiazol-4-yl)-2-hydroxyiminoacetamido group was located at the 7-position. Among them, there are some compounds which have a pyridinium group as the quaternary ammonium group and substituent groups on the pyridinium ring. These substituent groups are, however, extremely limited and these compounds are not always satisfactory in terms of the strength of antimicrobial or antibacterial activity and the broadness of antimicrobial or antibacterial spectrum.

Accordingly, it is an object of the present invention to provide a microbial or antibacterial agent, especially one which is effective against MRSA. The term MRSA used in the present specification is an abbreviation of methicillin-resistant Staphylococcus aureus.

The present invention thus provides novel antimicrobial or antibacterial agents and novel cephem compounds usable as an active principle of such antimicrobial or antibacterial agents, which satisfy the above object and other objects of the present invention which will become apparent from the description of the invention given hereinbelow.

In an aspect of the present invention there are provided some novel cephem compounds. In another aspect of the present invention, there are provided some novel antimicrobial or antibacterial agents containing, as an active ingredient, at least one of such cephem compounds.

The inventors of the present invention have examined various substituent groups to be located on the aforementioned pyridinium ring and found that compounds having a certain substituent group on the pyridinium ring can show an excellent antimicrobial activity, and a more excellent antibacterial activity especially on methicillin-resistant *Staphylococcus aureus* .

Accordingly, the present invention relates to cephem compounds represented by the followinq general formula (I) or physiologically or pharmaceutically acceptable salts thereof, and to antimicrobial or antibacterial agents which contain at least one of the compounds as an active ingredient.

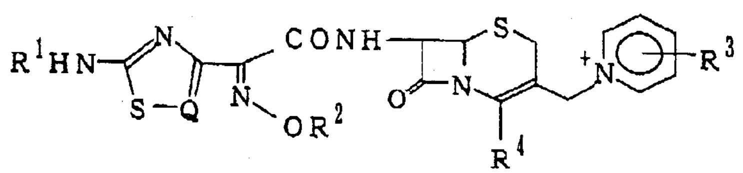

(I)

In the above formula, $R^1$ represents a hydrogen atom or an amino group protecting group; $R^2$ represents a hydrogen atom or a hydroxyimino group protecting group; $R^3$ is a 3- or 4-position substituent group on the pyridinium ring and represents a carbamoylmethyl group, a ureido group, thiazol group which may be substituted with an amino group, a thiadiazol group which may be substituted with an amino group, an oxadiazol group which may be substituted with an amino group, or a group represented by the following general formula (1):

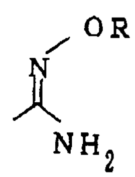

(1)

(wherein R represents a hydrogen atom or an alkyl group); $R^4$ represents a carboxyl or carboxylate group which may be protected; and Q represents CH or N.

As described above, the compounds of the present invention represented by the general formula (I) are cephem compounds which have a pyridinium methyl group with a specific substituent group at the 3- or 4-position of the pyridinium ring and, at the same time, an aminothiazolyloxyiminoacetamide or an aminothiadiazoloxyiminoacetamide at the 7-position.

In the aforementioned cephem compounds of the formula (I), the substituent group $R^3$ located on the pyridinium ring is one of the characteristic feature of the present invention, and such a substituent group creates new antimicrobial or antibacterial activity and spectrum.

Compounds of the present invention represented by the general formula (I) are all novel compounds, but can be produced in accordance with methods known per se.

That is, as shown hereinbelow, a compound of the formula (Ib) can be obtained by allowing a compound represented by the general formula (II) to react with NaI to convert the compound into its iodide, allowing the iodide to react with a substituent group-containing pyridine derivative represented by the general formula (III) to obtain a pyridinio group-containing compound represented by the general formula (Ia), and then eliminating the protective groups.

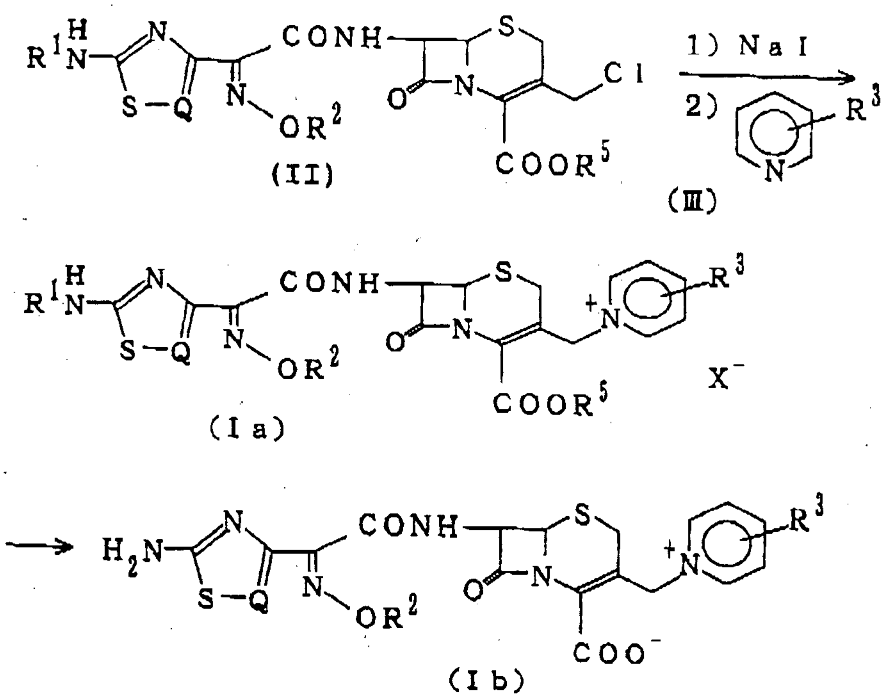


(II) (III) (Ia) (Ib)

In the above formulae, $R^1$, $R^2$, $R^3$ and Q have the same meanings, respectively, as described in the foregoing, and $R^5$ is a hydrogen atom or a protecting group of the carboxyl group, or $-COOR^5$ is $-COO^-$ as the whole, while $X^-$ does not exist when $-COOR^5$ is $-COO^-$, and $X^-$ is an inorganic or organic acid anion when $R^5$ is a hydrogen atom or a protecting group of the carboxyl group.

The protecting group ($R^1$) of the protected amino group means an easily detachable amino group-protecting group usually used in the cephalosporin derivatives chemistry, such as a formyl, a t-butoxycarbonyl, a benzyloxycarbonyl, a p-nitrobenzylcarbonyl, a trityl or the like group, which can be eliminated under relatively mild conditions. Examplea of the protecting group ($R^2$) of the protected hydroxyimino group include a trityl, a formyl, a tatrahydropyranyl, a chloroacetyl, a benzoyl and the like groups, which can be eliminated easily under reaction conditions known per se. The protecting group ($R^5$) of the protected carboxyl group means a β,β,β-trichloroethyl, a p-methoxybenzyl, a p-nitrobenzyl, a benzhydryl, a pivaloyloxymethyl or the like group, which can be eliminated easily.

The compounds of the present invention represented by the general formula (I) can also be produced for example by allowing a compound represented by the general formula (V) to react with a compound represented by the general formula (VI) and then eliminating the protecting groups as occasion demands, as in the following manner.

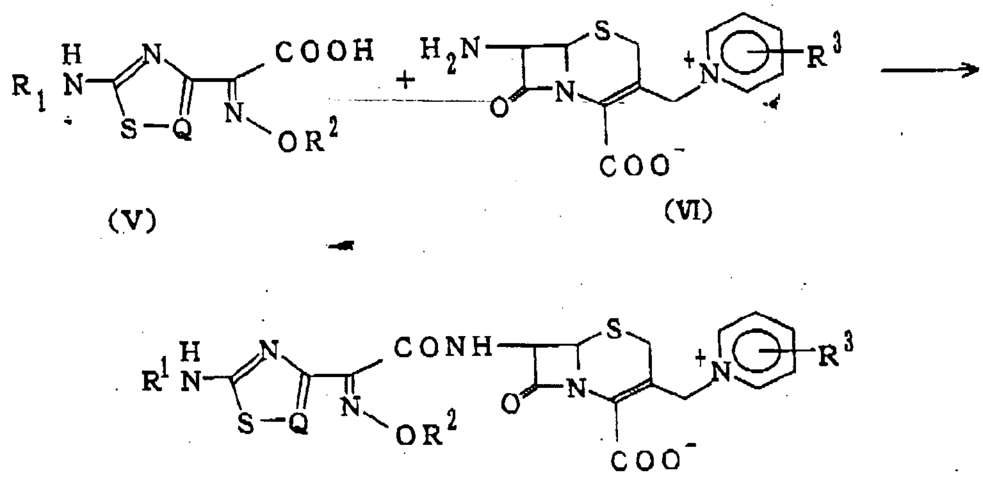

(V) (VI)

(I c)

In the above formulae, $R^1$, $R^2$, $R^3$ and Q have the same meanings, respectively, as described in the foregoing.

The above reaction will be described in greater detail, as follows. A compound of the formula (Ic) can be produced by allowing an acid halide obtained by treating a compound of the formula (V) with, e.g., phosphorus pentachloride, thionyl chloride, oxalyl chloride or the like, or a compound in the active form obtained by the treatment with Vilsmeier reagent, to react with a compound of the formula (VI) in a solvent which does not affect the reaction, such as dichloromethane, acetonitrile or the like, at a temperature of from -50 to 50°C, preferably from -10 to 30°C, in the presence of N,O-bis(trimethylsilyl) acetamide, triethylamine, sodium bicarbonate, propylene oxide or the like. Elimination reaction of protecting groups from a compound of the formula (Ic) can be carried out in a solvent which does not affect the reaction such as acetonitrile, dichloromethane or the like, or effected by treating the compound with an inorganic acid such as hydrochloric acid, an organic acid such as formic acid, trifluoroacetic acid or the like, or a mixture thereof without using a solvent. This reaction is carried out at a temperature of generally from -10 to 50°C, preferably from 0 to 30°C, and that in the presence of a scavenger such as anisole, thioanisole or the like.

The compounds of the present invention may be in the form of physiologically or pharmaceutically acceptable salts such as hydrachlorides, nitrates, sulfates, acetates, trifluoroacetates, tartarates,p-toluenesulfonates, methanesulfonates and the like.

The compounds of the present invention of the formula (I) and intermediates thereof contain an oxyimino group, and they have syn- and anti-isomers. The compounds of the present invention include of course both isomers.

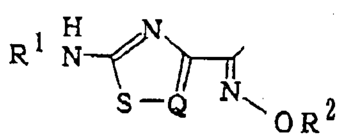

syn-isomer

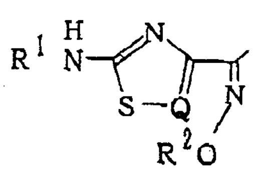

anti-isomer

In the above formulae, $R^1$, $R^2$ and Q have the same meanings, respectively, as described in the foregoing.

In the desired compounds of the formula (I) and their intermediates, the structure of the 2-amino-thiazol group moiety includes the two tautomers represented by the following equilibrium formula.

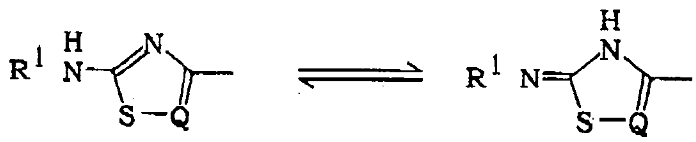

(A) (B)

In the above formula, $R^1$ and Q have the same meanings, respectively, as described in the foregoing.

Since it is well known that both of tautomers are present in the equilibrium and mutually changeable state, such isomers can be recognized as the substantially one and same compound. In consequence, insofar as the present invention is concerned, the aforementioned tautomeric groups contained in the desired compounds of the present invention and their starting compounds or intermediates is shown by the name of one of the tautomers, namely 2-aminothiazol group, and by the above formula (A) for the sake of convenience.

The present invention also provides an antimicrobial or antibacterial agent which contains at least one of the aforementioned cephem compounds represented by the general formula (I) as an active ingredient.

As will be described later in Test Example, the novel cephem compounds of the present invention have markedly broad antimicrobial or antibacterial Spectrum and show a surprisingly strong antimicrobial activity especially on methicillin-resistant *Staphylococcus aureus*. Also, the novel cephem compounds of the present invention have such an extremely low toxicity that acute toxicity was not found by the tests. These compounds can of course be used also as their physiologically or pharmaceutically acceptable salts.

Pharmaceutical compositions containing at least one of the compounds of the formula (I) and the salts thereof can be prepared in the usual way. The compositions contain at least one of the compounds of the formula (I) and the physiologically or pharmaceutically acceptable salts thereof and can be used in the form of conventional pharmaceutical preparations with pharmaceutically acceptable carriers such as organic or inorganic solid or liquid fillers incorporated therein, which are suitable for use in oral, parenteral or external administration. The pharmaceutical preparations may be in the form of either solid preparations such as tablets, granules, powders, capsules and the like or liquid preparations such as solutions, suspensions, syrups, emulsions and the like. As occasion demands, these preparations may contain isotonicity agents, solubilizing agents and other usually used additives. In other words, administration of the inventive preparations can be effected in accordance with the known antimicrobial agent administration methods.

Dose of the novel cephem compounds of the present invention varies depending on their type, age of individual patients, type of the diseases and the like, but they may be administered in a dose of generally from about 5 to 3,000 mg or higher per adult per day. Their administration may be carried out several times a day by dividing the daily dose into several portions.

Examples:

The following Examples and Test Example are provided to further illustrate the present invention, but the scope of the present invention is not limited by these examples as a matter of course.

Example 1: Synthesis of Compound 1 (7β-[2-(5-amino-1,2,4-thiadiazol-3-yl)-2-syn-hydroxyiminoacetamido]-3-(3-carbamoylmethylpyridinio)methyl-3-cephem-4-carboxylate).

(i) Synthesis of Compound Ia (p-methoxybenzyl 7β-[2-(5-t-butoxycarbonylamino-1,2,4-thiadiazol-3-yl)-2-syn-trityloxyiminoacetamido]-3-(3-carbamoylmethylpyridinio)methyl-3-cephem-4-carboxylate iodide).

845 mg of Compound 1b (p-methoxybenzyl 7β-[2-(5-t-butoxycarbonylamino-1,2,4-thiadiazol-3-yl)-2-syn-trityloxyiminoacetamido]-3-chloromethyl-3-cephem-4-carboxylate) was dissolved in 20 ml of acetone, and the resulting solution was added with 450 mg of NaI with stirring under ice-cooling and stirred at the same temperature for 10 minutes and then at room temperature for 1.5 hours. After distilling off the solvent, the residue was dissolved in ethyl acetate, and the solution was washed with an aqueous sodium chloride solution and dried on $Na_2SO_4$. After distilling off the solvent, the residue was dissolved in 10 ml of acetonitrile, added with 136 mg of 3-carbamoylmethylpyridine and then stirred at room temperature for 4 hours. The reaction solution was concentrated and added with isopropyl ether to effect crystallization, and the thus formed crystals were washed with isopropanol, collected by filtration and further washed with isopropyl ether to obtain 640 mg of the title compound.

IR ν max (nujol) $cm^{-1}$: 3342(sh), 3182, 1785, 1702, 1673, 1611, 1245, 1155, 960, 766, 704.

NMR δ ($CDCl_3$ + $CD_3OD$) ppm: 1.50 (9H, s), 3.15, 3.62 (2H, $AB_q$, J = 18), 3.76 (3H + 2H, br.s), 5.08 (1H,

d, J = 4.5), 5.20 (2H, s), 5.50, 5.95 (2H, $AB_q$, J = 14), 6.03 (1H, d = 4.5), 6.80 (2H, d, J = 9), 6.8 - 7.6 (17H, br. s), 7.6 - 9.1 (4H, m).

(ii) Synthesis of Compound 1.

590 mg of Compound 1a was dissolved in 2 ml of dichloromethane and, with stirring under ice-cooling, added with 0.4 ml of anisole and 1.2 ml of trifluoroacetic acid in this order, and the resulting mixture was stirred at the same temperature for 45 minutes and then at room temperature for 45 minutes. Thereafter, the solvent was distilled off, and 1.5 ml of trifluoroacetic acid and 0.5 ml of water in this order were added dropwise to the resulting residue with stirring under ice-cooling. The mixture was stirred at the same temperature for 20 minutes and then at room temperature for 2.5 hours, concentrated under reduced pressure and then mixed with isopropyl ether to obtain crystals, which were subsequently collected by filtration. The thus collected crystals were washed with isopropyl ether to obtain the trifluoroacetate. This was applied to a column packed with 100 ml of "HP20" (polystyrene resin manufactured by Mitsubishi Kasei Corp.) and developed with water and then with 20% aqueous solution of methanol, and the fraction containing the desired compound was concentrated and freeze-dried to obtain 41 mg of the title compound.

IR ν max (nujol) $cm^{-1}$: 3283 (sh), 3130, 1773, 1657, 1621, 1033, 854, 719.

NMR δ (DMSO-$d_6$ + $CD_3OD$) ppm: 3.26, 3.44 (2H, $AB_q$, J = 18), 3.67 (2H, s), 5.03 (1H, d, J = 4.5), 5.32, 5.48 (2H, $AB_q$, J = 14), 5.73 (1H, d, J = 4.5), 7.86 (1H, m), 8.23 (1H, m), 8.63 (1H, m), 8.69 (1H, br. s).

Example 2: Synthesis of Compound 2 (7β-[2-(5-amino-1,2,4-thiadiazol-3-yl)-2-syn-hydroxyiminoacetamido]-3-(3-ureidopyridinio)methyl-3-cephem-4-carboxylate).

(i) Synthesis of Compound 2a (p-methoxybenzyl 7β-[2-(5-t-butoxycarbonylamino-1,2,4-thiadiazol-3-yl)-2-syn-trityloxyiminoacetamido)-3-(3-ureidopyridinio)methyl-3-cephem-4-carboxylate iodide).

Using 880 mg of Compound 1b (p-methoxybenzyl 7β-[2-(5-t-butoxycarbonylamino-1,2,4-thiadiazol-3-yl)-2-syn-trityloxyiminoacetamido]-3-chloromethyl-3-cephem-4-carboxylate), 450 mg of NaI and 137 mg of 3-ureidopyridine, the procedure of Example 1 (i) was repeated to obtain 1.02 g of the title compound.

IR ν max (nujol) $cm^{-1}$: 3313, 3194, 1783, 1703, 1608, 1585, 1247, 1152, 986, 959, 759, 700.

NMR δ ($CDCl_3$ + $CD_3OD$) ppm: 1.50 (9H, s), 3.68 (3H, s), 3.16, 3.55 (2H, $AB_q$, J = 18), 5.10 (2H + 1H, br.s), 5.34 (2H, br.s), 5.95 (1H, d, J = 4.5), 6.66 (2H, d, J = 9), 6.7 - 7.5 (17H, br.s), 7.6 - 8.6 (3H, m), 9.13 (1H, br.s).

(ii) Synthesis of Compound 2.

Using 980 mg of Compound 2a, the procedure of Example 1 (ii) was repeated to obtain 104 mg of the title compound.

IR ν max (nujol) $cm^{-1}$: 3284, 3182, 1773, 1675, 1631, 1587, 1152, 1036, 1015, 805.

NMR δ (CMSO-$d_6$) ppm: 3.37, 3.52 (2H, $AB_q$, J = 18), 5.12 (1H, d, J = 4.5), 5.46 (2H, br. s), 5.80 (1H, d, d, J = 4.5, 9), 7.7 - 8.6 (3H, m), 9.18 (1H, br. s), 9.30 (1H, d, J = 9), 10.26 (1H, br.s).

Example 3: Synthesis of Compound 3 (7β-[2-(5-amino-1,2,4-thiadiazol-3-yl)-2-syn-hydroxyiminoacetamido]-3-[3-(2-atninothiazole-4-yl)pyridinio]methyl-3-cephem-4-carboxylate)

(i) Synthesis of Compound 3a (p-methoxybenzyl 7β-[2-(5-t-butoxycarbonylamino-1,2,4-thiadiazol-3-yl)-2-syn-trityloxyiminoacetamido]-3-[3-(2-aminothiazol-4-yl)pyridiniolrnethyl-3-cephem-4-carboxylate iodide).

Using 881 mg of Compound 1b (p-methoxybenzyl 7β-[2-(5-t-butoxycarbonylamino-1,2,4-thiadiazol-3-yl)-2-syn-trityloxyiminoacetamido]-3-chloromethyl-3-cephem-4-carboxylate), 450 mg of NaI and 171 mg of 3-(2-aminothiazol-4-yl)pyridine, the procedure of Example 1 (i) was repeated to obtain 930 mg of the title compound.

IR ν max (nujol) $cm^{-1}$: 3420, 3308 (sh), 3264, 3174, 1790, 1715, 1688, 1612, 1537, 1248, 1153, 962, 918, 762, 700.

NMR δ ($CDCl_3$ + $CD_3OD$) ppm: 1.47 (9H, s), 3.23, 3.70 (2H, $AB_q$, J = 18), 3.72 (3H, s), 5.03 (1H, d, J = 4.5), 5.16 (2H, s), 5.50, 5.67 (2H, $AB_q$, J = 13), 5.94 (1H, d, J = 4.5), 6.73 (2H, d, J = 8), 6.7 - 7.7 (15H + 2H, m), 7.7 - 9.5 (5H, m).

(ii) Synthesis of Compound 3.

The procedure of Example 1 (ii) was repeated using 900 mg of Compound 3a, the resulting trifluoroacetate was applied to a column packed with "HP20" and developed with 25% methanol which had been adjusted to pH 3 with hydrochloric acid, and then the fractions containing the desired compound were pooled, concentrated and freeze-dried to obtain 91 mg of the hydrochloride of the title compound.

IR ν max (nujol) $cm^{-1}$: 3273, 3175, 3101, 1778, 1666, 1634, 1526, 1153, 1018, 723, 673.

Example 4: Synthesis of Compound 4 (7β-[2-(5-amino-1,2,4-thiadiazol-3-yl)-2-syn-hydroxyiminoacetamido]-3-[4-thiazol-4-yl)pyridinio]methyl-3-cephem-4-carboxylate).

(i) Synthesis of Compound 4a (p-methoxybenzyl 7β-[2-(5-t-butoxycarbonylamino-1,2,4-thiadiazol-3-yl)-2-syn-trityloxyiminoacetamido]-3-[4-(thiazol-4-yl)pyridinio]methyl-3-cephem-4-carboxylateiodide).

Using 907 mg of Compound 1b (p-methoxybenzyl 7β-[2-(5-t-butoxycarbonylamino-1,2,4-thiadiazol-3-yl)-2-syn-trityloxyiminoacetamido]-3-chloromethyl-3-cephem-4-carboxylate), 460 mg of NaI and 162 mg of 4-(thiazol-4-yl)pyridine, the procedure of Example 1 (i) was repeated to obtain 936 mg of the title compound.

IR ν max (nujol) $cm^{-1}$: 3323, 3261(sh), 3196, 3057, 1790, 1717, 1684(sh), 1636, 1612, 1290, 1248, 1175, 1153, 827, 762, 700, 634.

NMR δ ($CDCl_3$ + $CD_3OD_3$) ppm: 1.48 (9H, s), 3.3 - 3.8 (2H, $AB_q$), 3.69 (3H, s), 5.03 (1H, d, J = 4.5), 5.12 (2H, s), 5.18 , 5.50 (2H, $AB_q$), 5.98 (1H, d, J = 4.5), 6.73 (2H, d, J = 9), 6.9 - 7.7 (15H + 2H, m), 8.32, 8.78 (4H, $AB_q$, J = 6), 8.39 (1H, d, J = 2), 8.76 (1H, d, J = 2).

(ii) Synthesis of Compound 4.

Using 896 mg of Compound 4a, the procedure of Example 1 (ii) was repeated to obtain 50 mg of the title compound.

IR υ max (nujol) $cm^{-1}$: 3150 (sh), 1778, 1673, 1633, 1526, 1204, 1155, 1030, 823, 770.

NMR δ (CMSO-$d_6$ + $CD_3OD$) ppm: 3.30, 3.53 (2H, $AB_q$, J = 18), 5.13 (1H, d, J = 4.5), 5.50 (2H, br.s), 5.82 (1H, d, J = 4.5), 8.5 - 9.5 (6H, m).

Example 5: Synthesis of Compound 5 (7β-[2-(5-amino-1,2,4-thiadiazol-3-yl)-2-syn-hydroxyiminoacetamido]-3-[3-(1,2,4-oxadiazol-5-yl)pyridinio]methyl-3-cephem-4-carboxylate).

(i) Synthesis of Compound 5a (p-methoxybenzyl 7β-[2-(5-t-butoxycarbonylamino-1,2,4-thiadiazol-3-yl)-2-syn-trityloxyiminoacetamido]-3-[3-(1,2,4-oxadiazol-5-yl)pyridinio]methyl-3-cephem-4-carboxylate iodide).

Using 1.760 g of Compound 1b (p-methoxybenzyl 7β-[2-(5-t-butoxycarbonylamino-1,2,4-thiadiazol-3-yl)-2-syn-trityloxyiminoacetamido]-3-chloromethyl-3-cephem-4-carboxylate), 900 mg of NaI and 270 mg of 3-(1,2,4-oxadiazol-5-yl)pyridine, the procedure of Example 1 (i) was repeated to obtain 1.031 g of the title compound.

IR ν max (KBr) $cm^{-1}$: 3057, 2972, 1792, 1717, 1612, 1541, 1516, 1448, 1371, 1248, 1151, 957, 762, 700.

NMR δ ($CDCl_3$ + $CD_3OD$) ppm: 1.53 (9H, s), 3.23 - 4.41 (2H + 3H), 5.14 (1H, d, J = 4.8), 5.24 (2H, s), 5.42 - 6.27 (2H, $AB_q$), 6.08 (1H, d, J = 4.8), 6.83 (2H, d, J = 8), 7.25 (17H, m), 7.96 - 8.40 (1H, m), 8.62 (1H, s), 8.80 - 9.38 (2H, m), 9.62 (1H, br.s).

(ii) Synthesis of Compound 5.

The procedure of Example 1 (ii) was repeated using 1.030 g of Compound 5a, 534 mg of the resulting trifluoroacetate was applied to a column packed with 140 ml of "HP20" and developed with water and then with a water-isopropanol mixed solvent system and then the fractions containing the desired compound were pooled, concentrated and freeze-dried to obtain 54 mg of the title compound.

IR ν max (KBr) $cm^{-1}$: 3396, 3308, 1774, 1670, 1616, 1522, 1508, 1460, 1394, 1354, 1171, 1148, 1016, 741, 677.

NMR δ (DMSO-$d_6$ + $CD_3OD$) ppm: 3.41 (1H, d, J = 18), 3.59 (1H, d, J = 18), 5.15 (1H, d, J = 4.5), 5.70 (2H, br.s), 5.84 (1H, d, J = 4.5), 8.07 - 8.59 (1H, m), 8.93 - 9.62 (2H, m), 9.20 (1H, s), 9.98 (1H, br.s).

Example 6: Synthesis of Compound 6 (7β-[2-(5-amino-1,2,4-thiadiazol-3-yl)-2-syn-hydroxyiminoacetamido]-3-[4-(1,2,4-oxadiazol-5-yl)pyridinio]methyl-3-cephem-4-carboxylate).

(i) Synthesis of Compound 6a (p-methoxybenzyl 7β-[2-(5-t-butoxycarbonylamino-1,2,4-thiadiazol-3-yl)-2-syn-trityloxyiminoacetamido]-3-[4-(1,2,4-oxadiazol-5-yl)pyridinio]methyl-3-cephem-4-carboxylate iodide).

Using 1.760 g of Compound 1b (p-methoxybenzyl 7β-[2-(5-t-butoxycarbonylamino-1,2,4-thiadiazol-3-yl)-2-syn-trityloxyiminoacetamido]-3-chloromethyl-3-cephem-4-carboxylate), 390 mg of NaI and 270 mg of 4-(1,2,4-oxadiazol-5-yl)pyridine, the procedure of Example 1 (i) was repeated to obtain 1.515 g of the title compound.

IR ν max (KBr) $cm^{-1}$: 3057, 2974, 1792, 1717, 1612, 1541, 1516, 1448, 1371, 1248, 1153, 1032, 957, 918, 860, 762, 700, 644.

NMR δ ($CDCl_3$ + $CD_3OD$) ppm: 1.54 (9H, s), 3.16 - 3.78 (2H, $AB_q$), 3.76 (3H, s), 5.15 (1H, d, J = 4.6), 5.22 (2H, s), 5.05 - 6.23 (2H, $AB_q$), 6.09 (1H, d, J = 4.6), 6.85 (2H, d, J = 8.4), 7.23 (17H, m), 8.56 (2H, d, J = 6.6), 8.72 (1H, s), 9.20 (2H, d, J = 6.6).

(ii) Synthesis of Compound 6.

Using 1.514 g of Compound 6a, the procedure of Example 5 (ii) was repeated to obtain 54 mg of the title compound.

IR ν max (KBr) $cm^{-1}$: 3307, 3185, 1773, 1670, 1616, 1522, 1458, 1395, 1362, 1292, 1240, 1150, 1065, 1016, 899, 853, 812, 750, 706.

Example 7: Synthesis of Compound 7 (7β-[2-(2-aminothiazol-4-yl)-2-syn-hydroxyiminoacetamido]-3-[3-(1,2,4-oxadiazol-5-yl)pyridinio]methyl-3-cephem-4-carboxylate).

(i) Synthesis of Compound 7a (p-methoxybenzyl 7β-[2-(2-tritylaminothiazol-4-yl)-2-syn-tritylhydroxyiminoacetamido]-3-[3-(1,2,4-oxadiazol-5-yl)pyridinio]methyl-3-cephem-4-carboxylate iodide).

Using 2.043 g of Compound 7b (p-methoxybenzyl 7β-[2-(2-tritylaminothiazol-4-yl)-2-syn-trityloxyiminoacetamido]-3-chloromethyl-3-cephem-4-carboxylate), 390 mg of NaI and 270 mg of 3-(1,2,4-oxadiazol-5-yl)pyridine, the procedure of Example 1

(i) was repeated to obtain 958 mg of the title compound.

IR ν max (KBr) $cm^{-1}$: 3057, 3022, 1794, 1720, 1674, 1628, 1612, 1514, 1491, 1448, 1248, 1219, 1175, 1034, 964, 752, 700.

NMR δ ($CDCl_3$ + $CD_3OD$) ppm: 3.53 (2H + 3H, br.s), 3.78 (3H, s), 5.20 (1H, d, J = 4.6), 5.27 (2H, s), 5.80 (2H, br.s), 6.06 (1H, d, J = 4.6), 6.53 (1H, s), 7.24 (32H, m), 8.52 (1H, m), 8.67 (1H, s), 8.88 (1H, m), 9.31 (1H, m), 9.74 (1H, br.s).

(ii) Synthesis of Compound 7.

Using 888 mg of Compound 7a, the procedure of Example 5

(ii) was repeated to obtain 53 mg of the title compound.

IR ν max (KBr) $cm^{-1}$: 1771, 1668, 1614, 1531, 1391, 1352, 1196, 1065, 818, 741.

NMR δ (DMSO-$d_6$ + $CD_3OD$) ppm: 3.52 (2H, br.s), 4.92 - 6.20 (2H, $AB_q$), 5.13 (1H, d, J = 4.3), 5.77 (1H, d, J = 4.3), 6.22 (1H, s), 8.36 (1H, m), 9.15 (1H + 1H, m + s), 9.49 (1H, m), 10.01 (1H, br.s).

Example 8: Synthesis of Compound 8 (7β-[2-(2-aminothiazol-4-yl)-2-syn-hydroxyiminoacetamido]-3-[4-(1,2,4-oxadiazol-5-yl)pyridinio]methyl-3-cephem-4-carboxylate).

(i) Synthesis of Compound 8a (p-methoxybenzyl 7β-[2-(2-tritylaminothiazol-4-yl)-2-syn-trityloxyiminoacetamido]-3-[4-(1,2,4-oxadiazol-5-yl)pyridinio]methyl-3-cephem-4-carboxylate iodide).

Using 2.043 g of Compound 1b (p-methoxybenzyl 7β-[2-(2-tritylaminothiazol-4-yl)-2-syn-trityloxyiminoacetamido]-3-chloromethyl-3-cephem-4-carboxylate), 390 mg of NaI and 270 mg of 4-(1,2,4-oxadiazol-5-yl)pyridine, the procedure of Example 1

(i) was repeated to obtain 1.047 g of the title compound.

IR ν max (KBr) $cm^{-1}$: 3057, 2964, 1792, 1674, 1514, 1491, 1448, 1248, 1217, 1177, 964, 752, 700, 635.

NMR δ ($CDCl_3$ + $CD_3OD$) ppm: 2.87 - 3.88 (2H, $AB_q$), 3.67 (3H, s), 5.10 (1H, d, J = 4.1), 5.21 (2H, s), 5.73 (2H, br.s), 5.98 (1H, d, J = 4.1), 6.48 (1H, s), 7.07 (32H, m), 8.52 (2H, d, J = 6.7), 8.68 (1H, s), 9.21 (2H, d, J = 6.7).

(ii) Synthesis of Compound 8.

Using 849 mg of Compound 8a, the procedure of Example 5 (ii) was repeated to obtain 43 mg of the title compound.

IR ν max (KBr) $cm^{-1}$: 3349, 1771, 1616, 1533, 1391, 1356, 1151, 814, 750, 706.

NMR δ (DMSO-$d_6$ + $CD_3OD$) ppm: 2.91 - 3.97 (2H, $AB_q$), 4.67 - 6.08 (2H, $AB_q$), 5.10 (1H, d, J = 4.1), 5.71 (1H, d, J = 4.1), 6.61 (1H, s), 8.70 (2H, d, J = 6.5), 9.24 (1H, s), 9.51 (2H, d, J = 6.5).

Example 9: Synthesis of Compound 9 (7β-[2-(2-aminothiazol-4-yl)-2-syn-hydroxyiminoacetamido]-3-[3-(thiazol-4-yl)pyridinio]methyl-3-cephem-4-carboxylate iodide).

(i) Synthesis of Compound 9a (p-methoxybenzyl 7β-[2-(2-tritylaminothiazol-4-yl)-2-syn-trityloxyiminoacetamido]-3-[3-(thiazol-4-yl)pyridinio]methyl-3-cephem-4-carboxylate iodide).

1.84 g of Compound 9b (p-methoxybenzyl 7β-[2-(2-tritylaminothiazol-4-yl)-2-syn-trityloxyiminoacetamido]-3-iodomethyl-3-cephem-4-carboxylate) was dissolved in 10 ml of acetonitrile and, with stirring and under ice-cooling, added with 324 mg of 3-(thiazol-4-yl)pyridine, and the resulting mixture was stirred at room temperature for 4 hours. After distilling off the solvent under reduced pressure, the thus obtained residue was mixed with ethyl acetate and stirred to remove materials dissolved in the ethyl acetate. Isopropyl alcohol was added to the resulting residue to effect crystallization, and the thus formed crystals were collected by filtration,

washed with isopropyl alcohol and then dried under reduced pressure to obtain 1.37 g of the title compound.

IR ν max (KBr) $cm^{-1}$: 1792, 1720, 1686, 1612, 1516, 1491, 1448, 1248, 1177, 1030, 962, 826, 754, 635.

NMR δ ($CDCl_3$ + $CD_3OD$) ppm: 3.1 - 4.0 (2H, $AB_q$), 3.69 (3H, s), 5.10 (1H, d, J = 5), 5.18 (2H, s), 5.69 (2H, br.s), 5.86 (1H, d, J = 5), 6.40 (1H, s), 6.72 (2H, d, J = 9), 7.04 (32H, br.s), 8.36 (1H, d, J = 2), 8.78 (1H, d, J = 2), 7.6 - 9.8 (4H, m).

(ii) Synthesis of Compound 9.

Using 1.30 g of Compound 9a, the procedure of Example 1 (ii) was repeated to obtain 212 mg of the title compound.

IR ν max (KBr) $cm^{-1}$: 1771, 1612, 1529, 1393, 1356, 1192, 1148, 988, 820.

NMR δ (DMSO-$d_6$ + $CD_3OD$) ppm: 3.26, 3.80 (2H, ABq, J = 17), 5.05 (1H, d, J = 5), 4.9 - 5.9 (2H, $AB_q$), 5.68 (1H, d, J = 5), 6.25 (1H, s), 8.39 (1H, d, J = 2), 9.09 (1H, d, J = 2), 7.9 - 9.9 (4H, m).

Example 10: Synthesis of Compound 10 (7β-[2-(2-aminothiazol-4-yl)-2-syn-hydroxyiminoacetamido]-3-[4-(thiazol-4-yl)pyridinio]methyl-3-cephem-4-carboxylate).

(i) Synthesis of Compound 10a (p-methoxybenzyl 7β-[2-(2-tritylaminothiazol-4-yl)-2-syn-trityloxyiminoacetamido]-3-[4-(thiazol-4-yl)pyridinio]methyl-3-cephem-4-carboxylate iodide).

Using 2.05 g of Compound 9b (p-methoxybenzyl 7β-[2-(2-tritylaminothiazol-4-yl)-2-syn-trityloxyiminoacetamido]-3-iodomethyl-3-cephem-4-carboxylate) and 324 mg of 4-(thiazol-4-yl)pyridine, the procedure of Example 9 (i) was repeated to obtain 982 mg of the title compound .

IR ν max (KBr) $cm^{-1}$: 1785, 1717, 1675, 1633, 1504, 1445, 1243, 1212, 1172, 1152, 1028, 960, 907, 827, 752, 700.

NMR δ ($CDCl_3$ + $CD_3OD$) ppm: 3.43 (2H, br.s), 3.73 (3H, s), 5.14 (1H, d, J = 5), 5.23 (2H, s), 5.61 (2H, br.s), 5.95 (1H, d, J = 5), 6.46 (1H, s), 6.80 (2H, d, J = 9), 7.14 (32H, m), 8.44 (2H, d, J = 6), 8.59 (1H, d, J = 2), 8.92 (1H, d, J = 2), 8.99 (2H, d, J = 6).

(ii) Synthesis of Compound 10.

Using 900 mg of Compound 10a, the procedure of Example 1 (ii) was repeated to obtain 129 mg of the title compound.

IR ν max (KBr) $cm^{-1}$: 1762, 1630, 1528, 1350, 1150, 1040, 823.

NMR δ (DMSO-$d_6$ + $CD_3OD$) ppm: 3.73 (2H, br.s), 5.09 (1H, d, J = 5), 5.47 (2H, br.s), 5.72 (1H, d, J = 5), 6.60 (1H, s), 8.56 (2H, d, J = 6), 8.93 (1H, d, J = 2), 9.22 (2H, d, J = 6), 9.28 (1H, d, J = 2).

Example 11: Synthesis of Compound 11 (7β-[2-(2-aminothiazol-4-yl)-2-syn-hydroxyiminoacetamido]-3-[4-(5-amino-1,2,4-thiadiazol-3-yl)pyridinio]methyl-3-cephem-4-carboxylate).

(i) Synthesis of Compound 11a (p-methoxybenzyl 7β-[2-(2-tritylaminothiazol-4-yl)-2-syn-trityloxyiminoacetamido]-3-[4-(5-amino-1,2,4-thiadiazol-3-yl)pyridinio]methyl-3-cephem-4-carboxylate iodide).

1.89 g of Compound 9b (p-methoxybenzyl 7β-[2-(2-tritylaminothiazol-4-yl)-2-syn-trityloxyiminoacetamido]-3-iodomethyl-3-cephem-4-carboxylate) and 285 mg of 4-(5-amino-1,2,4-thiadiazol-3-yl)pyridine were dissolved in 10 ml of dimethylformamide with stirring under ice-cooling, and the resulting solution was stirred at room temperature for 2 hours. 75 ml of isopropyl alcohol was added to the solution to effect crystallization. After cooling, the thus formed crystals were collected by filtration, washed with isopropyl alcohol, and then dried to obtain 1.37 g of the title compound.

IR ν max (KBr) $cm^{-1}$: 1794, 1719, 1665, 1636, 1612, 1516, 1472, 1448, 1369, 1248, 1177, 1032, 962, 827, 752, 700, 635, 401.

NMR δ ($CDCl_3$ + $CD_3OD$) ppm: 3.1 - 4.0 (2H, $AB_q$), 3.69 (3H, s), 5.12 (1H, d, J = 5), 5.17 (1H, s), 5.58 (2H, br.s), 5.82 (1H, d, J = 5), 6.43 (1H, s), 6.73 (2H, d, J = 9), 7.06 (30H + 2H, br.s), 8.38 (2H, d, J = 6), 8.93 (2H, d, J = 6).

(ii) Synthesis of Compound 11

Using 1.33 g of Compound 11a, the procedure of Example 1 (ii) was repeated to obtain 153 mg of the title compound.

IR ν max (KBr) $cm^{-1}$: 1771, 1636, 1529, 1474, 1371, 1192, 1144, 1047, 988, 692.

NMR δ (DMSO-$d_6$ + $CD_3OD$) ppm: 3.29, 3.66 (2H, $AB_q$, J = 18), 5.14 (1H, d, J = 5), 5.1 - 5.9 (2H, $AB_q$), 5.80 (1H, d, J = 5), 6.58 (1H, s), 8.45 (2H, d, J = 6), 9.09 (2H, d, J = 6).

Example 12: Synthesis of Compound 12 (7β-[2-(2-aminothiazol-4-yl)-2-syn-hydroxyiminoacetamido]-3-[3-(5-amino-1,2,4-thiadiazol-3-yl)pyridinio]methyl-3-cephem-4-carboxylate).

(i) Synthesis of Compound 12a (p-methoxybenzyl 7β-[2-(2-tritylaminothiazol-4-yl)-2-syn-trityloxyiminoacetamido]-3-[3-(5-amino-1,2,4-thiadiazol-3-yl)pyridinio]methyl-3-cephem-4-carboxylate iodide).

Using 1.52 g of a compound 9b (p-methoxybenzyl 7β-[2-(2-tritylaminothiazol-4-yl)-2-syn-trityloxyiminoacetamido]-3-iodomethyl-3-cephem-4-carboxylate) and 243 mg of 3-(5-amino-1,2,4-thiadiazole), the procedure of Example 11 (i) was repeated to obtain 945 mg of the title compound.

IR ν max (KBr) $cm^{-1}$: 1794, 1719, 1666, 1612, 1516, 1448, 1383, 1248, 1177, 1099, 1032, 964, 827, 754, 700, 635.

NMR δ ($CDCl_3$ + $CD_3OD$) ppm: 3.0 - 3.8 (2H, $AB_q$), 3.72 (3H, s), 5.13, (1H, d, J = 5), 5.19 (2H, s), 5.66 (2H, br.s), 5.92 (1H, d, J = 5), 6.43 (1H, s), 6.74 (2H, d, J = 9), 7.12 (30H + 2H, br.s), 7.7 - 9.5 (4H, m).

(ii) Synthesis of Compound 12.

Using 930 mg of Compound 12a, the procedure of Example 1 (ii) was repeated to obtain 82 mg of the title compound.

IR ν max (KBr) $cm^{-1}$: 1771, 1616, 1528, 1481, 1379, 1290, 1184, 1138, 723, 419.

NMR δ (DMSO-$d_6$ + $CD_3OD$) ppm: 3.35, 3.85 (2H, $AB_q$, J = 17), 5.10 (1H, d, J = 5), 4.9 - 5.9 (2H, $AB_q$), 5.75 (1H, d, J = 5), 6.61 (1H, s), 7.1 - 9.8 (4H, m).

Example 13: Synthesis of Compound 13 (7β-[2-(2-aminothiazol-4-yl)-2-syn-hydroxyiminoacetamido]-3-[4-(5-amino-1,2,4-oxadiazol-3-yl)pyridinio]methyl-3-cephem-4-carboxylate).

(i) Synthesis of Compound 13a (p-methoxybenzyl 7β-[2-(2-tritylaminothiazol-4-yl)-2-syn-trityloxyiminoacetamido]-3-[4-(5-amino-1,2,4-oxadiazol-3-yl)pyridinio]methyl-3-cephem-4-carboxylate iodide).

Using 1.67 g of Compound 9b (p-methoxybenzyl 7β-[2-(2-tritylaminothiazol-4-yl)-2-syn-trityloxyiminoacetamido]-3- iodomethyl-3-cephem-4-carboxylate) and 259 mg of 4-(5-amino-1,2,4-oxadiazol-3-yl)pyridine, the procedure of Example 11 (i) was repeated to obtain 1.26 mg of the title compound.

IR ν max (KBr) $cm^{-1}$: 1794, 1720, 1659, 1612, 1516, 1493, 1448, 1410, 1248, 1177, 1032, 962, 827, 762, 700, 635, 419.

NMR δ ($CDCl_3$ + $CD_3OD$) ppm: 3.1 - 4.1 (2H, $AB_q$), 3.63 (3H, s), 5.08 (1H, d, J = 5), 5.11 (2H, s), 5.58 (2H, br.s), 5.88 (1H, d, J = 5), 6.36 (1H, s), 6.67 (2H, d, J = 8), 7.00 (30H + 2H, br.s), 8.17 (2H, d, J = 6), 9.93 (2H, d, J = 6).

(ii) Synthesis of Compound 13.

Using 1.23 g of Compound 13a, the procedure of Example 1 (ii) was repeated to obtain 158 mg of the title compound.

IR ν max (KBr) $cm^{-1}$: 1771, 1663, 1616, 1531, 1412, 1356, 1138, 814, 764, 419.

NMR δ (DMSO-$d_6$ + $CD_3OD$) ppm: 3.17, 3.65 (2H, $AB_q$, J = 17), 5.06 (1H, d, J = 5), 5.1 - 5.9 (2H, $AB_q$), 5.71 (1H, d, J = 5), 6.55 (1H, s), 8.32 (2H, d, J = 6), 9.27 (2H, d, J = 6).

Example 14: Synthesis of Compound 14 (7β-[2-(2-aminothiazol-4-yl)-2-syn-hydroxyiminoacetamido)-3-[3-(5-amino-1,2,4-oxadiazol-3-yl)pyridinio]methyl-3-cephem-4-carboxylate).

(i) Synthesis of Compound 14a (p-methoxybenzyl 7β-[2-(2-tritylaminothiazol-4-yl)-2-syn-trityloxyiminoacetamido)-3-[3-(5-amino-1,2,4-oxadiazol-3-yl)pyridinio]methyl-3-cephem-4-carboxylate iodide).

Using 1.64 g of Compound 9b (p-methoxybenzyl 7β-[2-(2-tritylaminothiazol-4-yl)-2-syn-trityloxyiminoacetamido-3-iodomethyl-3-cephem-4-carboxylate) and 243 mg of 3-(5-amino-1,2,4-oxadiazol-3-yl)pyridine, the procedure of Example 11 (i) was repeated to obtain 1.59 g of the title compound.

IR ν max (KBr) $cm^{-1}$; 1792, 1719, 1659, 1516, 1448, 1412, 1248, 1177, 1099, 1032, 964, 827, 700, 635.

NMR δ ($CDCl_3$ + $CD_3OD$) ppm: 3.1 - 4.0 (2H, $AB_q$), 3.70 (3H, s), 5.09 (1H, d, J = 5), 5.15 (2H, s), 5.67 (2H, br.s), 5.92 (1H, d, J = 5), 6.40 (1H, s), 6.72 (2H, d, J = 9), 7.09 (30H + 2H, br.s), 7.8 - 9.3 (4H, m).

(ii) Synthesis of Compound 14.

Using 1.58 g of Compound 14a, the procedure of Example 1 (ii) was repeated to obtain 103 mg of the title compound.

IR ν max (KBr) $cm^{-1}$: 1773, 1663, 1616, 1533, 1414, 1352, 1178, 818, 764.

NMR δ (DMSO-$d_6$ + $CD_3OD$) ppm: 3.17, 3.64 (2H, $AB_q$, J = 17), 5.04 (1H, d, J = 5), 5.1 - 6.0 (2H, $AB_q$), 5.71 (1H, d, J = 5), 6.54 (1H, s), 7.9 - 9.8 (4H, m).

Example 15: Synthesis of Compound 15 (7β-[2-(2-aminothiazol-4-yl)-2-syn-hydroxyiminoacetamido]-3-[4-(2-amino-1,3,4-oxadiazol-5-yl)pyridinio]methyl-3-cephem-4-carboxylate).

(i) Synthesis of Compound 15a (p-methoxybenzyl 7β-[2-(2-tritylaminothiazol-4-yl)-2-syn-trityloxyiminoacetamido]-3-[4-(2-amino-1,3,4-oxadiazol-5-yl)pyridinio]methyl-3-cephem-4-carboxylate iodide).

Using 1.67 g of Compound 9b (p-methoxybenzyl 7β-[2-(2-tritylaminothiazol-4-yl)-2-syn-trityloxyiminoacetamido]-3-iodomethyl-3-cephem-4-carboxylate) and 259 mg of 4-(2-amino-1,3,4-oxadiazol-5-yl)pyridine, the procedure of Example 11 (i) was repeated to obtain 1.26 g of the title compound.

IR ν max (KBr) $cm^{-1}$: 1797, 1720, 1628, 1516, 1448, 1248, 1177, 1034, 964, 829, 754, 700, 635.

NMR δ ($CDCl_3$ + $CD_3OD$)ppm : 3.1 - 4.1 (2H, $AB_q$), 3.68 (3H, s), 5.19 (2H, s), 5.22 (1H, d, J = 5), 5.56, 2H, br.s), 5.87 (1H, d, J = 5), 6.50 (1H, s), 6.79 (2H, d, J = 8), 7.16 (30H + 2H, br.s), 8.19 (2H, d, J = 6), 8.88 (2H, d, J = 6).

(ii) Synthesis of Compound 15.

Using 1.00 g of Compound 15a, the procedure of Example 1 (ii) was repeated to obtain 145 mg of the title compound.

IR ν max (KBr) $cm^{-1}$: 1771, 1636, 1394, 1356, 1047, 851, 741, 681.

Reference Example 1: Synthesis of 3-(5-amino-1,2,4-oxadiazol-3-yl)pyridine.

14.5 g of 3-cyanopyridine, 50 ml of water, 9.68 g of hydroxylamine hydrochloride and 7.38 g of $Na_2CO_3$ were put in an autoclave and, under sealed condition, stirred for 8 hours with heating at an outer water bath temperature of 70°C. After cooling, the reaction mixture was concentrated, dissolved in 100 ml of ethanol by heating, and spontaneously cooled. The inorganic materials were removed by filtration, and the filtrate was then concentrated. Chloroform was added to the concentrate to effect crystallization, and the thus formed crystals were collected by filtration and dried to obtain 17.0 g of nicotinamidoxime nicotinate. A 5.2 g portion of this compound was dissolved in 38 ml of methanol in which 4.44 g of KOH had been dissolved, 5.0 g of BrCN was added in small portions with stirring to the resulting solution at a temperature of 0°C or lower, and then the mixture was stirred for 2 hours until its temperature reached the room temperature. Thereafter, the thus formed crystals were collected by filtration, treated with 40 ml of hot water and then, after spontaneous cooling, collected by filtration and dried. The title compound was obtained in an amount of 1.47 g.

IR ν max (KBr) $cm^{-1}$: 1682, 1600, 1482, 1401, 1149, 1123, 1005, 970, 893, 812, 758, 682, 625.

NMR δ (DMSO-$d_6$) ppm: 7.42 (1H, d.d, J = 8,5), 7.92 (2H, br.s), 8.11 (1H, d.t, J = 8,2), 8.58 (1H, d.d, J = 5,2), 9.93 (1H, d, J = 2).

Reference Example 2: Synthesis of 4-(5-amino-1,2,4-oxadiazol-3-yl)pyridine.

17.1 g of 4-cyanopyridine, 70 ml of water, 11.4 g of hydroxylamine hydrochloride and 8.7 g of $Na_2CO_3$ were stirred in an autoclave for 8 hours with heating at 70°C and, after cooling, the resulting reaction mixture was mixed with 60 ml of water, heated again and then spontaneously cooled to obtain 20.8 g of isonicotinamidoxime. A 5.5 g portion of this compound was dissolved in 50 ml of methanol containing 4.7 g of KOH, 5.5 g of BrCN dissolved in 15 ml of ether was added dropwise to the resulting solution with stirring at a temperature of 0°C or lower, and then the mixture was stirred for 1 hour at 0°C to collect the formed crystals by filtration. Thereafter, the thus collected crystals were washed with 1/2 N KOH aqueous solution and then with water, dissolved in 40 ml of dimethylformamide by heating. After cooling, the formed precipitates were collected by filtration, washed with methanol and dried. The title compound was obtained in an amount of 1.40 g.

IR ν max (KBr) $cm^{-1}$: 1685, 1612, 1490, 1404, 1314, 1060, 1010, 1001, 893, 835, 675, 600.

NMR δ (DMSO-$d_6$) ppm: 7.70 (2H, d.d, J = 4,2), 7.97 (2H, br.s), 8.63 (2H, d.d, J = 4,2).

Reference Example 3: Synthesis of 4-(1,2,4-oxadiazol-5-yl)pyridine.

10.0 g of isonicotinamide was dissolved in 200 ml of dichloromethane, mixed with 20 ml of dimethylformamide dimethylacetal, and then stirred at room temperature for 18 hours, followed by concentration. The concentrate was mixed with carbon tetrachloride, and the thus formed crystals were collected by filtration to obtain 13.85 g of dimethylaminomethyleneamide isonicotinate. A 1.77 g portion of this compound was dissolved in 8 ml of methanol, and the solution was added with 1.695 g of hydroxylamine-O-sulfonic acid and 1.56 ml of pyridine in this order and then stirred at room temperature for 18 hours. After concentration, the concentrate was made alkaline, with $K_2CO_3$, extracted with chloroform and dried on $Na_2SO_4$, and then the solvent was evaporated to obtain 389 mg of the title compound.

IR ν max (KBr) $cm^{-1}$: 3053, 1579, 1545, 1457, 1411, 1335, 1296, 1247, 1170, 1090, 952, 939, 869, 836, 744, 719, 693, 633.

NMR δ (DMSO) ppm: 7.94 (2H, d.d, J = 1.6, 4.3), 8.53, (1H, s), 8.80 (2H, d.d, J = 1.6, 4.3).

Reference Example 4: Synthesis of 3-(5-amino-1,2,4-thiadiazol-3-yl)pyridine.

9.0 g of 3-cyanopyridine was dissolved in 75 ml of chloroform, and the solution was added with 5.7 ml of ethanol and then cooled to -5°C. Into this was bubbled dry chlorine gas to saturation. After 24 hours of standing at 0°C, the reaction solution was added to 100 g of ice water containing 12 g of NaOH to separate the chloroform layer. This was washed with water and dried on $K_2CO_3$, and then the solvent was removed by distillation to obtain 11.9 g of an imino ether compound in an oily form. This was dissolved in 29 ml of ethanol and 8 ml of water, and the solution was mixed with 1.8 g of ammonium chloride and stirred for 4 hours with heating at 70°C. The reaction solution was mixed with activated carbon and filtered, and the filtrate was mixed with 300 ml of acetone to collect the formed crystals by filtration, thereby obtaining 4.75 g of the amidino compound. A 4.6 g portion of this compound was dissolved in 66 ml of methanol, and mixed with 10.2 ml of triethylamine, and then 4.9 g of bromine was added dropwise to the thus prepared mixture with stirring under cooling at -5°C. To this was added dropwise 45 ml of methanol in which 2.84 g of KSCN had been dissolved, followed by 2 hours of stirring until the mixture was warmed up to room temperature. Crystals thus formed were collected by filtration, heated with 40 ml of 1 N NaOH, collected again by filtration after cooling, washed with water and then dried to obtain 4.6 g of the title compound.

NMR δ (DMSO-$d_6$) ppm: 7.39 (1H, d.d, J = 8.5), 8.04 (2H, br.s), 8.30 (1H, d.t, J = 8,2), 8.55 (1H, d.t, J = 5,2), 9.17 (1H, d, J = 2).

Reference Example 5: Synthesis of 4-(5-amino-1,2,4-thiadiazol-3-yl)pyridine.

Using 9.0 g of 4-cyanopyridine, the procedure of Reference Example 4 was repeated to obtain 2.77 g of the title compound.

IR ν max (KBr) $cm^{-1}$: 1640, 1605, 1533, 1445, 1412, 1364, 1083, 1057, 1000, 833, 805, 700, 668.

NMR δ (DMSO-$d_6$) ppm: 7.84 (2H d.d, J = 4,2), 8.03 (2H, br.s), 8.58 (2H, d.d, J = 4,2).

Reference Example 6: Synthesis of 3-(thiazol-4-yl)pyridine.

4.73 g of formamide was dissolved in 10 ml of dioxane, and the solution was mixed with 6.67 g of $P_2S_5$ and subjected to 1.5 hours of reflux. After cooling, the reaction solution was mixed with 100 ml of methanol and 4.21 g of 3-bromoacetylpyridine hydrobromate in this order and subjected to 1 hour of reflux. Thereafter, the solvent was evaporated and the resulting residue was mixed with 70 ml of water, made alkaline with 8N NaOH, and then extracted with chloroform. The extract was dried on $Na_2SO_4$, concentrated, applied to a column packed with 75 g of silica gel and then developed with a mixed solvent system of chloroform:methanol = 20:1 to collect and concentrate the fractions containing the desired compound, thereby obtaining 963 mg of the title compound.

NMR δ ($CDCl_3$) ppm: 7.27 (1H, d.d, J = 8,5), 7.57 (1H, d, J = 2), 8.15 (1H, d.t, J = 8,2), 8.49 (1H, d.d, J = 5,2), 8.83 (1H, d, J = 2), 9.07 (1H, d, J = 2).

Reference Example 7: Synthesis of 4-(thiazol-4-yl)pyridine.

Using 4.5 g of formamide, 6.7 g of $P_2S_5$ and 3.78 g of 4-bromoacetylpyridine hydrobromide, the procedure of Reference Example 6 was repeated to obtain 881 mg of the title compound.

IR ν max (KBr) $cm^{-1}$: 1594, 1472, 1421, 1209, 1221, 1182, 1047, 989, 908, 885, 829, 756, 681, 472.

NMR δ ($CDCl_3$) ppm: 7.66 (2H, d, J = 2), 7.69 (2H, d.d, J = 4,2), 8.57 (2H, d.d, J = 4,2), 8.80 (1H, d, J =2).

Reference Example 8: Synthesis of 4-(2-amino-1,3,4-oxadiazol-5-yl)pyridine.

5.5 g of isonicotinic acid hydrazide was dissolved in 50 ml of methanol containing 4.7 g of KOH and cooled to -5°C. With stirring, to this was added dropwise 5.5 g of BrCN dissolved in 15 ml of dry ether, followed by 1 hour of stirring at 0°C. Crystals thus formed were collected by filtration, mixed thoroughly with 20 ml of water, collected again by filtration, and then dried to obtain 1.57 g of the title compound.

IR ν max (KBr) $cm^{-1}$: 1653, 1585, 1567, 1412, 1299, 1222, 1125, 1038, 1900, 827, 735, 682, 533.

NMR δ (CDSO-$d_6$) ppm: 7.40 (2H, br, s), 7.62 (2H, d.d, J = 4,2), 8.63 (2H, d.d, J = 4,2).

Reference Example 9: Synthesis of 3-(1,2,4-oxadiazol-5-yl)pyridine.

10.0 g of nicotinamide was dissolved in 200 ml of dichloromethane, and the solution was mixed with 20 ml of dimethylformamide dimethylacetal and stirred overnight at room temperature. After concentration, the resulting residue was dissolved in 150 ml of methanol. To the resulting solution was subsequently added dropwise 14.15 g of hydroxylamine-O-sulfonate and 12.5 ml of pyridine in this order, followed by 48 hours of stirring at room temperature. The reaction mixture was concentrated, mixed with 150 ml of water, alkalinized with $K_2CO_3$ and then extracted with dichloromethane. This was washed with water and dried on $Na_2SO_4$, the solvent was distilled off, and then the residue was solidified with hexane and collected by filtration. This was applied to a column packed with 100 g of $SiO_2$ and developed with a mixed solvent system of chloroform:methanol = 9:1 to obtain 2.44 g of the title compound.

IR ν max (KBr) $cm^{-1}$: 3084, 1600, 1580, 1550, 1450, 1424, 1333, 1286, 1235, 1159, 1121, 1024, 963, 952, 929, 869, 816, 738, 699, 632, 625.

NMR δ ($CDCl_3$) ppm: 7.45 (1H, d.d.d, J = 1.0, 4.6, 8.0), 8.38 (1H, d.d.d, J = 1.6, 2.2, 8.0), 8.51 (1H, s), 8.78 (1H, d.d, J = 1.6, 4.6), 9.33 (1H, d.d, J = 1.0, 2.2).

Reference Example 10: Synthesis of 3-(1,2,4-oxadiazol-5-yl)pyridine.

10.0 g of nicotinamide was dissolved in 200 ml of dichloromethane, and the solution was mixed with 20 ml of dimethylformamide dimethylacetal and stirred overnight at room temperature. After concentration, the resulting residue was dissolved in 150 ml of methanol. To the resulting solution was subsequently added dropwise 14.15 g of hydroxylamine-O-sulfonate and 12.5 ml of pyridine in this order, followed by 48 hours of stirring at room temperature. The reaction mixture was concentrated, mixed with 150 ml of water, alkalinized with $K_2CO_3$, and then extracted with dichloromethane. This was washed with water and dried on $Na_2SO_4$, the solvent was distilled off, and then the residue was solidified with hexane and collected by filtration. This was applied to a column packed with 100 g of $SiO_2$ and developed with a mixed solvent system of chloroform:methanol = 9:1 to obtain 2.44 g of the title compound.

IR ν max (KBr) $cm^{-1}$: 3084, 1600, 1580, 1550, 1450, 1424, 1333, 1286, 1235, 1159, 1121, 1024, 963, 952, 929, 869, 816, 738, 699, 632, 625.

NMR δ ($CDCl_3$) ppm: 7.45 (1H, d. d. d, J = 1.0, 4.6, 8.0), 8.38 (1H, d. d. d, J = 1.6, 2.2, 8.0), 8.51 (1H, s), 8.78 (1H, d. d. J = 1.6, 4.6), 9.33 (1H, d. d. J = 1.0, 2.2).

Example 16: Synthesis of Compound A (7β-[2-(2-aminothiazol-4-yl)-2-syn-hydroxyiminoacetamido]-3-[4-(3-aminotriazol-5-yl) pyridinio]methyl-3-cephem-4-carboxylate).

The reaction sequence in this example is shown below. The compound numbers used are for this example only.

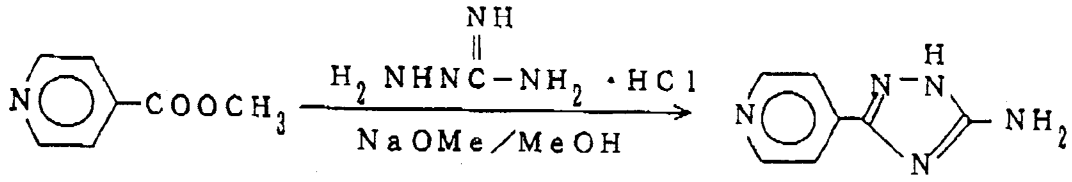


5

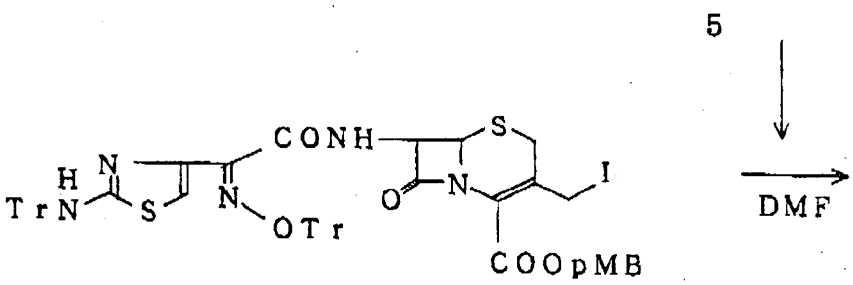


4

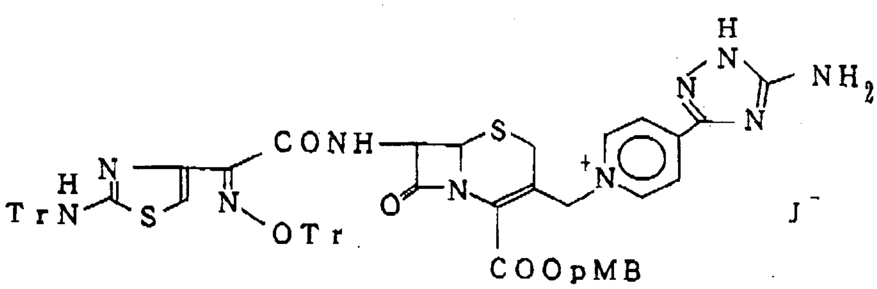

6

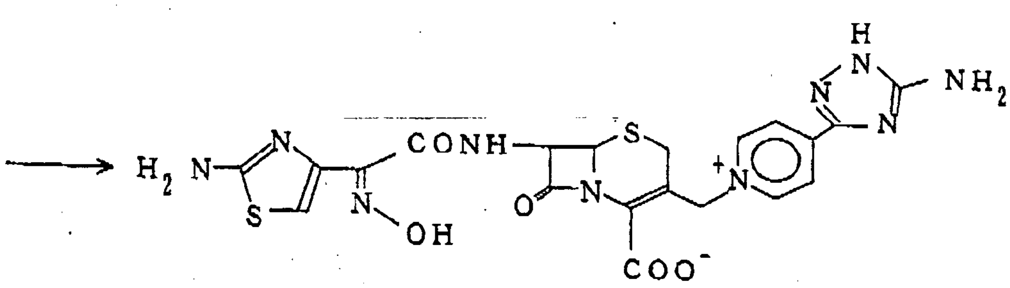

A

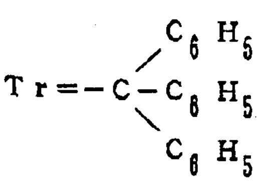

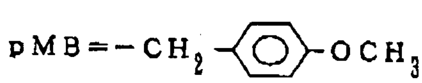

(i) Synthesis of Compound 5 (4-(3-aminotriazol-5-yl)pyridine.

6.89 g of nicotinic acid methyl ester and 5.53 g of aminoguanidine hydrochloride were added to 25 ml of methanol. To the mixture was subsequently added dropwise 19.3 g of 28% sodium methylate solution, and the resulting mixture was stirred at room temperature for 1 hour and then heated under reflux for 4 hours. The reaction solution was neutralized by the dropwise addition of 8.3 ml of 6 N HCl and evaporated to dryness, the resulting residue was dissolved in 30 ml of water by heating, treated with activated carbon, filtered and cooled, and then the thus precipitated crystals were collected by filtration and dried to obtain 3.29 g (41%) of Compound 5.

IR ν max (KBr) $cm^{-1}$: 1663, 1607, 1491, 1420, 1350, 1312, 1157, 1051, 1001, 837, 756.

NMR δ (DMSO-$d_6$) ppm: 5.16 (2H, s), 7.74 (2H, d.d. J = 4,2), 8.52 (2H, d.d, J = 4,2), 12,39 (1H, br, s).

(ii) Synthesis of Compound 6 (p-methoxybenzyl 7β-[2-(2-tritylaminothiazol-4-yl)-2-syn-trityloxyiminoacetamido]-3-[4-(3-aminotriazol-5-yl)pyridinio]methyl-3-cephem-4-carboxylate iodide).

8 ml of DMF was added under cooling to 1.671 g of Compound 4 (p-methoxybenzyl 7β-[2-(2-tritylaminothiazol-4-yl)-2-syntrityloxyiminoacetamido]-3-iodomethyl-3-cephem-4-carboxylate) and 266 mg of Compound 5, the resulting mixture was stirred at room temperature for 3 hours, DMF was distilled off under reduced pressure, the resulting residue was dispersed in 80 ml of isopropyl alcohol to effect crystallization and, after cooling, the thus formed crystals were collected by filtration, washed with isopropyl alcohol and dried to obtain 1.45 g (76%) of Compound 6.

IR ν max (KBr) $cm^{-1}$: 1792, 1720, 1634, 1516, 1491, 1448, 1248, 1177, 1099, 1032, 962, 827, 752, 700, 635.

NMR δ ($CDCl_3$ + $CD_3OD$) ppm: 3.19, 3.6 - 4.0 (2H, $AB_q$, J = 18), 3.67 (3H, s), 5.05 (1H, d, J = 5), 5.16 (2H, s), 5.52 (2H, br.s), 5.88 (1H, d, J = 5), 6.39 (1H, s), 6.72 (2H, d, J = 9), 7.08 (30H, br.s), 7.18 (2H, d, J = 9), 8.12 (2H, d, J = 6), 8.80 (2H, d, J = 6).

(iii) Synthesis of Compound A.

1.42 g of Compound 6 was dissolved in 7.1 ml of dichloromethane, and 1.42 ml of anisole and 2.84 ml of trifluoroacetic acid were added in this order dropwise under cooling to the thus prepared solution. The resulting mixture was stirred at room temperature for 2 hours followed by distillation removal of the solvent, and ice-cooled. 9.5 ml of trifluoroacetic acid and 3.2 ml of water in this order were added dropwise to the ice-cooled residue. The resulting mixture was stirred at room temperature for 4 hours and concentrated under reduced pressure, the thus obtained residue was solidified by addition of ether, the thus solidified material was collected by filtration, dissolved in methanol-hydrochloric acid, applied to a column packed with 225 ml of "HP20" and developed with water and the 20% aqueous methanol solution in this order, and then resulting fractions containing the desired compound were concentrated and freeze-dried to obtain 198 mg (33%) of the title compound, i.e., Compound A.

IR ν max (KBr) $cm^{-1}$: 1771, 1636, 1537, 1493, 1393, 1354, 988, 752.

NMR δ (DMSO-$d_6$ + $CD_3OD$) ppm: 3.21, 3.63 (2H, $AB_q$, J = 18), 5.11 (1H, d, J = 5), 5.1 - 5.8 (2H, $AB_q$), 5.76 (1H, d, J = 5), 6.59 (1H, s), 8.27 (2H, d, J = 6), 9.06 (2H, d, J = 6).

Example 17: Synthesis of Compound B (7β-[2-(2-aminothiazol-4-yl)-2-syn-hydroxyiminoacetamido]-3-[4-(1,2,4-oxadiazol-3-yl)pyridinio]methyl-3-cephem-4-carboxylate).

The reaction sequence in this example is shown below. The compound numbers used are for this example only.

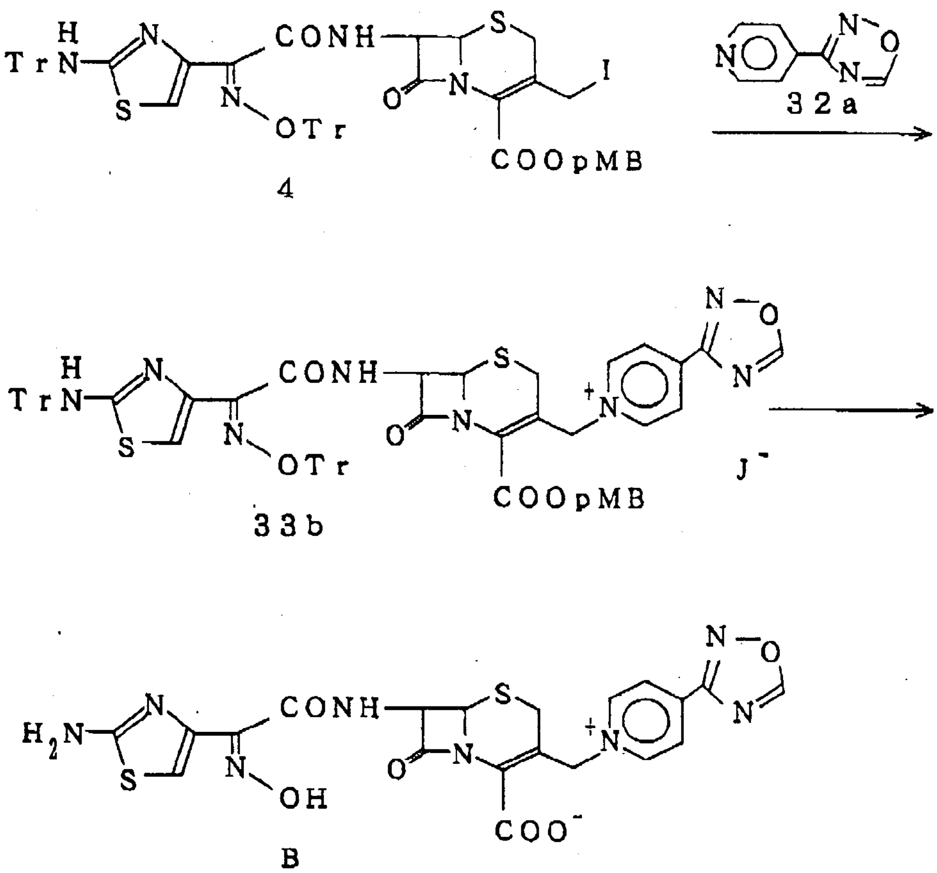

4

32a

33b

B

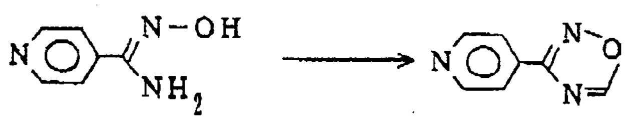

20a

32a

(i) Synthesis of Compound 33b.

1.727 g of Compound 4 and 250 mg of Compound 32a (4-(1,2,4-oxadiazol-3-yl)pyridine) were used and treated in the same manner as in the synthesis of Compound 6 in Example 16 to obtain 1.771 g (91%) of Compound 33b.

IR ν max (KBr) $cm^{-1}$: 1792, 1717, 1684, 1612, 1516, 1448, 1248, 1177, 1105, 1032, 962, 750, 700, 635.

NMR δ ($CDCl_3$ + $CD_3OD$) ppm: 3.1 - 4.1 (2H, $AB_q$), 3.84 (3H, s), 5.14 (1H, d, J = 5), 5.15 (2H, s), 5.64 (2H, br.s), 5.89 (1H, d, J = 5), 6.40 (1H, s), 6.70 (2H, d, J = 9), 7.11 (30H + 2H, s), 8.42 (2H, d, J = 6), 8.87 (1H, s), 9.11 (2H, d, J = 6).

(ii) Synthesis of Compound B.

1.760 g of Compound 33b was used and treated in the same manner as in the synthesis of Compound A in Example 16 to obtain 139 mg (19%) of Compound B.

IR ν max (KBr) $cm^{-1}$: 1773, 1645, 1616, 1526, 1360, 1192, 1157, 1109, 814, 708.

NMR δ (DMSO-$d_6$ + $CD_3OD$) ppm: 3.32, 3.83 (2H, $AB_q$, J = 18), 5.06 (1H, d, J = 5), 5.1 - 5.9 (2H, $AB_q$), 5.71 (1H, d, J = 5), 6.53 (1H, s), 8.52 (2H, d, J = 6), 9.30 (2H, d, J = 6), 9.65 (1H, s).

(iii) Synthesis of Compound 32a.

A mixture consisting of 3.76 g of Compound 20a (4-(1-amino-1-hydroxyiminomethyl)pyridine) and 30 ml of ethyl orthoformate was stirred for 15 hours with heating while distilling off the formed alcohol at an outer temperature of 160°C, the ethyl orthoformate was then distilled off under reduced pressure, the thus obtained residue was dissolved in 50 ml of ethyl acetate by heating, the thus prepared solution was treated with activated carbon and filtered while hot, the resulting filtrate was cooled by the addition of 50 ml of hexane, and then crystals thus formed and additional crystals formed by concentration of the mother liquid were collected by filtration and dried to obtain a total of 2.17 g (54%) of Compound 32a.

NMR δ (DMSO-$d_6$) ppm: 7.83 (2H, d.d, J = 4,2), 8.65 (2H, d.d, J = 4,2), 9.60 (1H, s).

Example 18: Synthesis of compound C (7β-[2-(2-aminothiazol-4-yl)-2-syn-hydroxyiminoacetsmido]-3-[4-(1,3,4-oxadiazol-2-yl)pyridinio]methyl-3-cephem-4-carboxylate).

The reaction sequence in this example is shown below. The compound numbers used are for this example only.

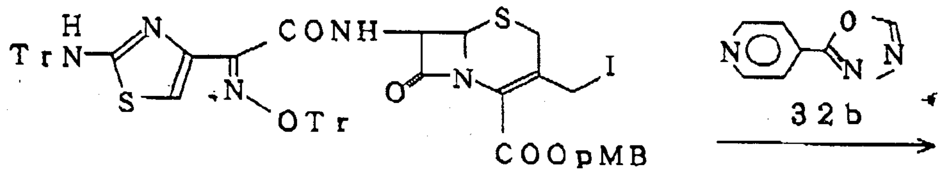

32b

4

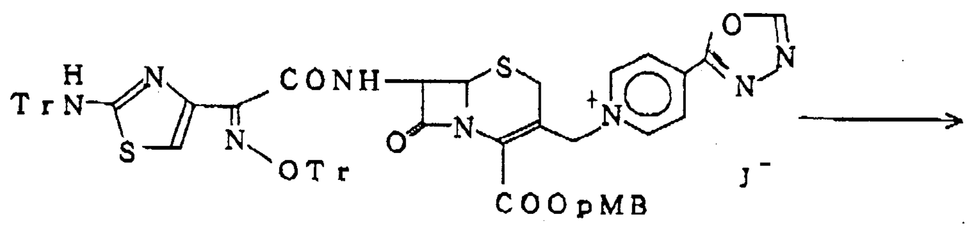

33c

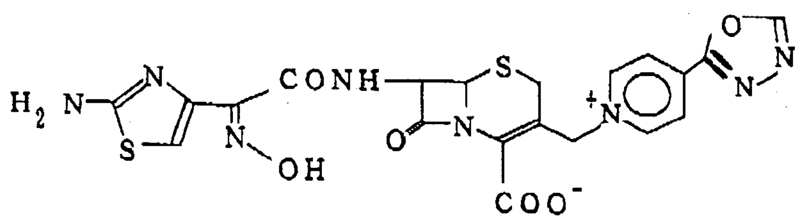

C

(i) Synthesis of Compound 33c.

1.727 g of Compound 4 and 250 mg of Compound 32b (4-(1,3,4-oxadiazol-2-yl)pyridine) were used and treated in the same manner as in the synthesis of Compound 6 in Example 16 to obtain 1.653 g (85 %) of Compound 33c.

IR ν max (KBr) $cm^{-1}$: 1792, 1717, 1682, 1643, 1612, 1516, 1448, 1248, 1177, 1032, 961, 754, 700, 634.

NMR δ ($CDCl_3$ + $CD_3OD$) ppm: 3.1 - 4.1 (2H, $AB_q$), 3.65 (3H, s), 5.11 (1H, d, J = 5), 5.14 (2H, s), 5.59 (2H, br.s), 5.86 (1H, d, J = 5), 6.38 (1H, s), 6.68 (2H, d, J = 9), 7.07 (30H + 2H, s), 8.33 (2H, d, J = 6), 8.77 (1H, s), 9.04 (2H, d, J = 6).

(ii) Synthesis of Compound C.

A 1.480 g portion of the Compound 33c was used and treated in the same manner as in the synthesis of Compound A in Example 16 to obtain 78 mg (13%) of Compound C.

IR ν max (KBr) $cm^{-1}$: 1773, 1666, 1533, 1393, 1192, 1128, 1065, 988, 814.

NMR δ (DMSO-$d_6$ + $CD_3OD$) ppm: 2.98, 3.60 (2H, $AB_q$, J = 17), 5.12 (1H, d, J = 5), 5.2 - 5.9 (2H, $AB_q$), 5.76 (1H, d, J = 5), 6.58 (1H, s), 8.05 (1H, s), 8.35 (2H, d, J = 6), 9.18 (2H, d, J = 6).

(iii) Synthesis of Compound 32b.

A mixture consisting of 5.5 g of isonicotinic acid hydrazide and 40 ml of ethyl orthoformate was stirred for 6 hours with heating while distilling off the formed alcohol at an outer temperature of 150 to 160°C. The ethyl orthoformate was distilled off, the thus obtained residue was dissolved in 50 ml of ethyl acetate by heating, treated with activated carbon and spontaneously cooled, and then the crystals thus formed and the additional crystals formed by concentration of the mother liquor were collected by filtration to obtain a total of 5.78 g (97%) of Compound 32b.

NMR δ (DMSO-$d_6$) ppm: 7.81 (2H, d.d, J = 4, 2), 8.93 (2H, d.d, J = 4, 2), 9.27 (1H, s).

Example 19: Synthesis of Compound D (7β-[2-(2-aminothiazol-4-yl)-2-syn-hydroxyiminoacetamido]-3-[3-(1-amino-1-hydroxyiminomethyl)pyridinio]methyl-3-cephem-4-carboxylate).

The reaction sequence in this example is shown below. The compound numbers used are for this example only.

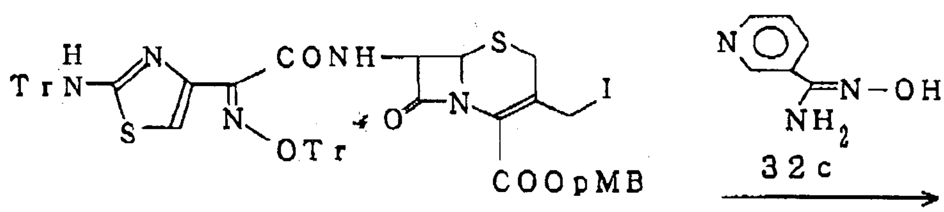


4

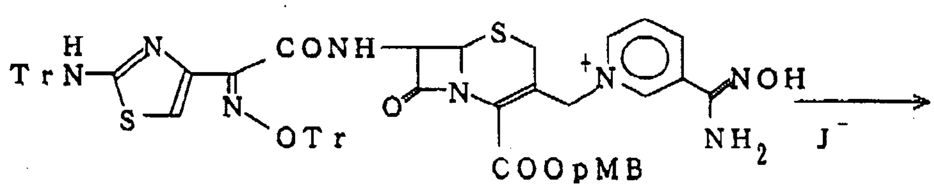

33d

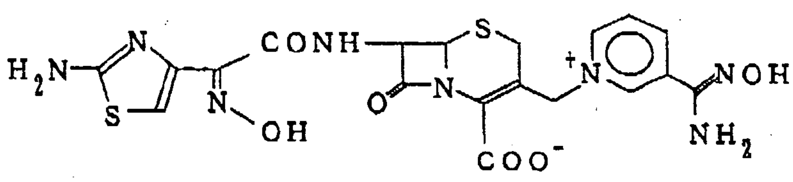

D

(i) Synthesis of Compound 33d.

1.727 g of Compound 4 and 298 mg of Compound 32c (3-(1-amino-1-hydroxyiminomethyl)pyridine) were taken and treated in the same manner as in the synthesis of Compound 6 in Example 16 to obtain 1.652 g (85%) of Compound 33d.

IR ν max (KBr) $cm^{-1}$: 1792, 1719, 1653, 1516, 1448, 1389, 1248, 1177, 1032, 964, 827, 754, 700, 635.

NMR δ ($CDCl_3$ + $CD_3OD$) ppm: 3.0 - 4.1 (2H, $AB_q$), 3.69 (3H, s), 5.05 (1H, d, J = 5), 5.14 (2H, s), 5.52 (2H, br.s), 5.86 (1H, d, J = 5), 6.37 (1H, s), 6.71 (2H, d, J = 9), 7.07 (30H + 2H, s), 7.5 - 9.3 (4H, m).

(ii) Synthesis of Compound D.

A 1.620 g portion of the Compound 33d was taken and treated in the same manner as in the synthesis of Compound A in Example 16 to obtain 122 mg (18 %) of Compound D.

IR ν max (KBr) $cm^{-1}$: 1771, 1653, 1612; 1533, 1393, 1192, 817, 685.

NMR δ (DMSO-$d_6$ + $CD_3OD$) ppm: 3.13, 3.60 (2H, $AB_q$, J = 18), 5.03 (1H, d, J = 5), 5.0 - 5.9 (2H, $AB_q$), 5.68 (1H, d, J = 5), 6.54 (1H, s), 7.8 - 9.5 (4H, m).

(iii) Synthesis of Compound 32c.

14.5 g of 3-cyanopyridine, 50 ml of water, 9.68 g of hydroxylamine hydrochloride and 7.38 g of $Na_2CO_3$ were stirred for 8 hours in an autoclave with heating at an outer temperature of 70°C, the resulting reaction mixture was concentrated and heated in 100 ml of ethanol, the inorganic materials precipitated after spontaneous cooling were removed by filtration, the ethanol was evaporated, the residue was dispersed in 70 ml of chloroform to effect crystallization, and then the thus formed crystals were collected by filtration and dried to obtain 17.0 g (89%) of Compound 32c.

Example 20: Synthesis of Compound E (7β-[2-(2-aminothiazol-4-yl)-2-syn-hydroxyiminoacetamido]-3-[4-(1-amino-1-hydroxyiminomethyl)pyridinio]methyl-3-cephem-4-carboxylate).

The reaction sequence in this example is shown below. The compound numbers used are for this example only.

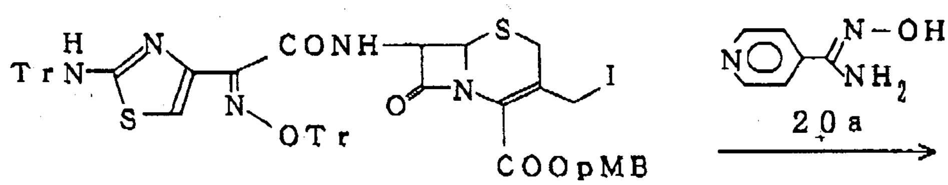


4

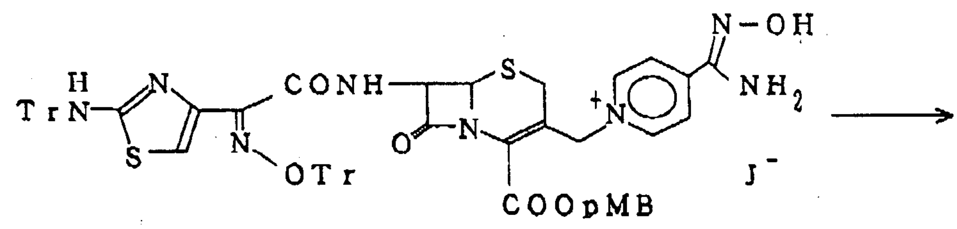

33e

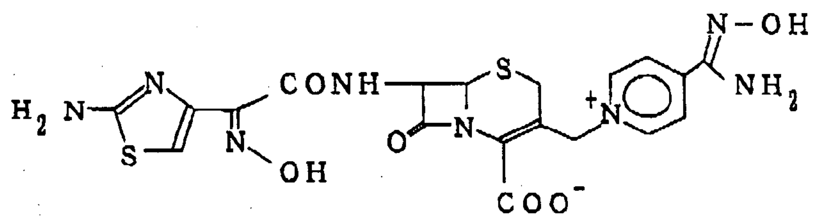

E

(i) Synthesis of Compound 33e.

1.700 g of Compound 4 and 293 mg of Compound 20a (4-(1-amino-1-hydroxyiminomethyl)pyridine) were taken and treated in the same manner as in the synthesis of Compound 6 in Example 16 to obtain 1.533 g (80%) of Compound 33e.

IR ν max (KBr) $cm^{-1}$: 1792, 1720, 1655, 1624, 1516, 1448, 1339, 1248, 1177, 1032, 964, 829, 754, 700, 635.

NMR δ ($CDCl_3$ + $CD_3OD$) ppm: 3.0 - 4.1 (2H, $AB_q$), 3.70 (3H, s), 5.08 (1H, d, J = 5), 5.16 (2H, s), 5.49 (2H, br.s), 5.89 (1H, d, J = 5), 6.40 (1H, s), 6.73 (2H, d, J = 9), 7.08 (30H + 2H, s), 8.01 (2H, d, J = 6), 8.75 (2H, d, J = 6).

(ii) Synthesis of Compound E.

A 1.500 g portion of the Compound 33e was taken and treated in the same manner as in the synthesis of Compound A in Example 16 to obtain 161 mg (26%) of Compound E.

IR ν max (KBr) $cm^{-1}$: 1771, 1653, 1628, 1531, 1393, 1130, 968.

NMR δ (DMSO-$d_6$ + $CD_3OD$) ppm: 3.09, 3.55 (2H, $AB_q$, J = 18), 5.02 (1H, d, J = 5), 5.0 - 5.7 (2H, $AB_q$), 5.60 (1H, d, J = 5), 6.53 (1H, s), 8.13 (2H, d, J = 6), 9.02 (2H, d, J = 6).

Example 21: Synthesis of Compound F (7β-[2-(2-aminothiazol-4-yl)-2-syn-hydroxyiminoacetamido]-3-[4-(thiazol-2-yl)pyridinio]methyl-3-cephem-4-carboxylate).

The reaction sequence in this example is shown below. The compound numbers used are for this example only.

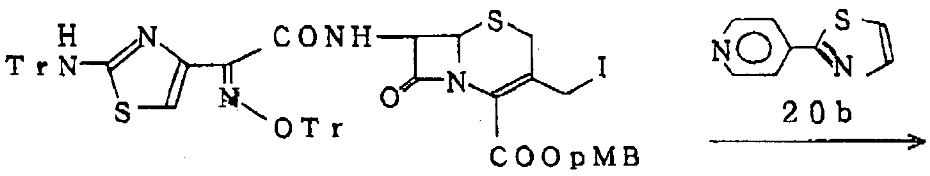


4

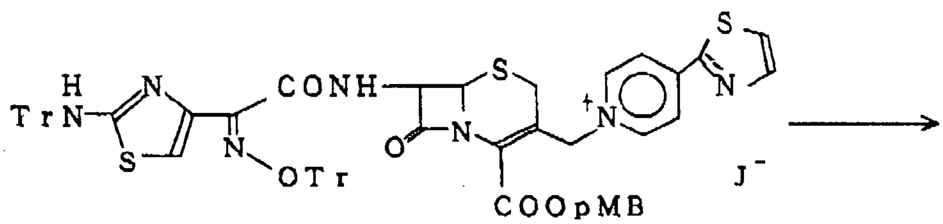

33f

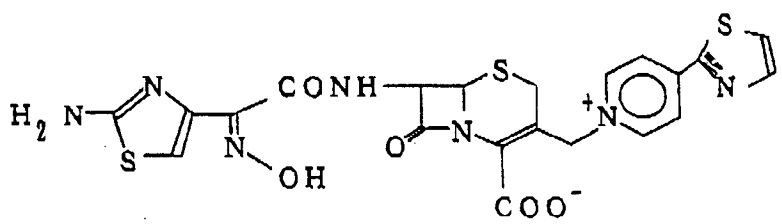

F

(i) Synthesis of Compound 33f.

1.671 g of Compound 4 and 292 mg of Compound 20b (4-(thiazol-2-yl)pyridine) were taken and treated in the same manner as in the synthesis of Compound 6 in Example 16 to obtain 1.389 g (73 %) of Compound 33f.

IR ν max (KBr) $cm^{-1}$: 1792, 1720, 1682, 1636, 1516, 1448, 1389, 1248, 1177, 1032, 964, 829, 754, 700, 635.

NMR δ ($CDCl_3$ + $CD_3OD$) ppm: 3.0 - 4.0 (2H, $AB_q$), 3.65 (3H, s), 5.12 (1H, d, J = 5), 5.15 (2H, s), 5.58 (2H, br.s), 5.87 (1H, d, J = 5), 6.39 (1H, s), 6.71 (2H, d, J = 9), 7.09 (30H, s), 7.20 (2H, d, J = 9), 7.70 (1H, d, J = 3), 7.97 (1H, d, J = 3), 8.26 (2H, d, J = 6), 8.94 (2H, d, J = 6).

(ii) Synthesis of Compound F.

A 1.350 g portion of the Compound 33f was taken and treated in the same manner as in the synthesis of Compound A in Example 16 to obtain 164 mg (29 %) of Compound F.

IR ν max (KBr) $cm^{-1}$: 1774, 1636, 1522, 1481, 1391, 997, 633.

NMR δ (DMSO-$d_6$ + $CD_3OD$) ppm: 3.13, 3.60 (2H, $AB_q$, J = 17), 5.00 (1H, d, J = 5), 5.0 - 5.8 (2H, $AB_q$), 5.64 (1H, d, J = 5), 6.47 (1H, s), 7.96 (2H, s), 8.32 (2H, d, J = 6), 9.09 (2H, d, J = 6).

(iii) Synthesis of Compound 20b.

13.7 g of methyl isonicotinate and 10.5 g of 2-aminoacetaldehyde dimethylacetal were heated for 2 hours while distilling off the formed methanol at an outer temperature of 50°C, and the resulting reaction mixture was applied to a column packed with 300 g of $SiO_2$ and developed with a mixed solvent system of chloroform:methanol = 30:1 to 20:1 to obtain 10.73 g (51%) of N-(2-dimethoxyethyl)isonicotinamide [NMR δ ($CDCl_3$) ppm: 3.37 (6H, s), 3.56 (2H, d, J = 5), 4.42 (1H, t, J = 5), 7.00 (1H, br.s), 7.48 (2H, d.d, J = 4, 2), 8.50 (2H, d.d, J = 4, 2)]. A 10.5 g portion of this compound and 15.6 g of $P_2S_5$ were heated for 1 hour at an outer temperature of 110°C, the mixture was dissolved in 500 ml of 1 N KOH, extracted with chloroform and treated with activated carbon, the chloroform was evaporated, and the residue was applied to a column packed with 150 g of $SiO_2$ and developed with a mixed solvent system of chloroform:methanol = 30:1 to obtain 2.75 g (34%) of Compound 20b.

IR ν max (KBr) $cm^{-1}$: 1595, 1502, 1475, 1414, 1323, 1273, 1256, 1215, 1151, 1072, 995, 988, 878, 824, 768, 748, 698, 637, 465.

NMR δ ($CDCl_3$) ppm: 7.28 (1H, d, J = 3), 7.63 (2H, d.d, J = 4, 2), 7.77 (1H, d, J = 3), 8.51 (2H, d.d, J = 4,

2).

Example 22: Synthesis of Compound G (7β-[2-(2-aminothiazol-4-yl)-2-syn-hydroxyiminoacetamido]-3-[4-(thiazol-5-yl)pyridinio]methyl-3-cephem-4-carboxylate).

The reaction sequence in this example is shown below. The compound numbers used are for this example only.

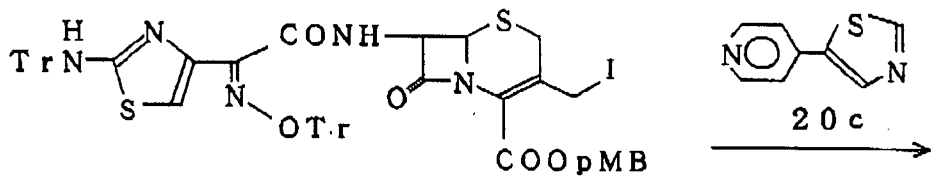


4

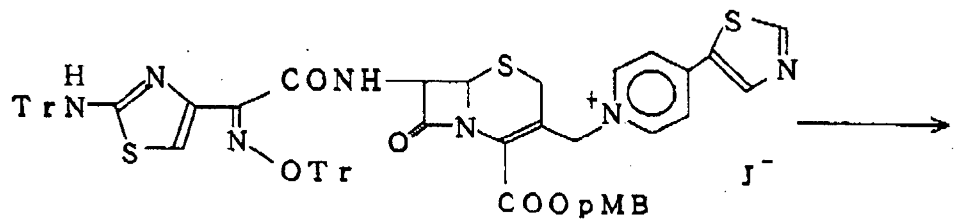

33g

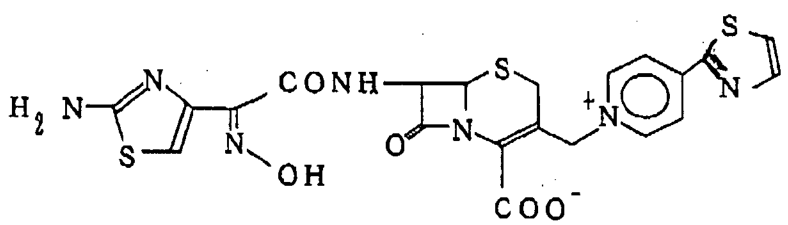

G

(i) Synthesis of Compound 33g.

1.671 g of Compound 4 and 292 mg of Compound 20c (4-(thiazol-5-yl)pyridine) were dissolved in 7 ml of acetonitrile and 3 ml of DMF with stirring under ice cooling, the thus prepared solution was stirred at room temperature for 3 hours, the solvent was distilled off under reduced pressure, the resulting residue was suspended in 70 ml of isopropyl alcohol, and then the thus formed crystals were collected by filtration and dried to obtain 1.636 g (86 %) of Compound 33g.

IR ν max (KBr) $cm^{-1}$: 1792, 1720, 1676, 1636, 1531, 1516, 1491, 1448, 1248, 1177, 1032, 964, 829, 754, 700, 635.

NMR δ ($CDCl_3$ + $CD_3OD$) ppm: 3,31 (1H, $AB_q$, J = 18), 3.6 - 4.0 (1H, $AB_q$), 3.70 (3H, s), 5.11 (1H, d, J = 5), 5.16 (2H, s), 5.56 (2H, br.s), 5.80 (1H, d, J = 5), 6.41 (1H, s), 6.75 (2H, d, J = 9), 7.14 (30H, s), 7.19 (2H, d, J = 9), 8.00 (2H, d, J = 6), 8.52 (1H, s), 8.91 (2H, d, J = 6), 8.98 (1H, s).

(ii) Synthesis of Compound G.

A 1.620 g portion of the Compound 33g was taken and treated in the same manner as in the synthesis of Compound A in Example 16 to obtain 159 mg (23 %) of Compound G.

IR ν max (KBr) $cm^{-1}$: 1773, 1636, 1533, 1464, 1393, 1356, 1236, 1157, 1126, 986, 812.

NMR δ (DMSO-$d_6$ + $CD_3OD$) ppm: 3.21, 3.67 (2H, $AB_q$, J = 17), 5.12 (1H, d, J = 5), 5.23, 5.63 (2H, $AB_q$), J = 12), 6.60 (1H, s), 8.29 (2H, d, J = 6), 8.79 (1H, s), 9.15 (2H, d, J = 6), 9.30 (1H, s).

(iii) Synthesis of Compound 20c.

12.1 g of 4-acetylpyridine was dissolved in 60 ml of methanol, added with 7.1 g of $NH_2OH$-HCl and stirred at room temperature for 1 hour, the reaction solution was concentrated under reduced pressure and further dried under reduced pressure, the thus obtained residue was added with 15 ml of triethylamine and 100 ml of

pyridine and then, under cooling, with 22.9 g of p-toluenesulfonic acid chloride and stirred at room temperature for 24 hours, the reaction mixture was added with stirring to 600 ml of ice water and cooled, the thus formed precipitates were collected by filtration, dissolved in 100 ml of methanol, treated with activated carbon, mixed with 300 ml of water and cooled, and then the thus formed, precipitates were collected by filtration and dried to obtain 22.55 g (78%) of 4-(1-tosyloxyimino)ethylpyridine.

The thus obtained compound was dissolved in 57 ml of anhydrous ethanol and added dropwise to 45 ml of anhydrous ethanol containing 3.0 g of metallic potassium in a stream of Ar with stirring under cooling at 0°C, the resulting mixture was stirred at 10°C for 2.5 hours, the thus precipitated crystals were removed by filtration, the resulting mother liquor was concentrated and mixed with 150 ml of ether, the crystals further precipitated were removed by filtration, the resulting filtrate was mixed with 12.9 g of 99% formic acid and concentrated, to the thus obtained residue was added dropwise a solution which had been prepared by 30 minutes of stirring of a mixture of 30 ml of 99% formic acid and 30 ml of acetic acid anhydride, the resulting mixture was stirred at an outer temperature of 30 to 40°C for 2.5 hours followed by distillation removal of the solvent under reduced pressure, and then the resulting residue was applied to a column packed with 200 g of $SiO_2$ and developed with a mixed solvent system of chloroform:methanol = 30:1 to 20:1 to obtain 6.24 g (37.8%) of 4-(1-diethoxy-2-formylamino)ethylpyridine. A 6.16 g portion of this compound and 8.6 g of $P_2S_5$ were heated for 1 hour at an outer temperature of 120°C, dissolved in 300 ml of 1 N KOH, extracted with chloroform, dried on $Na_2SO_4$, treated with activated carbon and subjected to distillation removal of the solvent, and then the resulting residue was applied to a column packed with 150 g of $SiO_2$ and developed with a mixed solvent system of chloroform:methanol = 30:1 to 20:1 to obtain 1.044 g (25%) of Compound 20c.

IR $\nu$ max (KBr) $cm^{-1}$: 1595, 1551, 1414, 1387, 1306, 1232, 1117, 993, 876, 818, 696, 619, 561, 473.

NMR $\delta$ ($CDCl_3$) ppm: 7.28 (2H, d.d, J = 4.2), 8.08 (1H, s), 8.45 (2H, d.d, J = 4.2), 8.68 (1H, s).

Test Example:

Antimicrobial activity of the compounds of the present invention were measured using CTM (cefotiam) and FMOX (flomoxef) as control drugs in accordance with the standard method of Japan Society of Chemotherapy. Of the 25 standard strains used in this test, *Staphylococcus aureus* 167 and *Staphylococcus aureus* 195 belong to MRSA.

The results are shown in the following Tables 1 and 2. In these tables, drugs tested are shown by the corresponding examples in which they were synthesized.

Table 1: MIC (μg/ml) on standard strains

| Strains tested | Drugs tested | | | | | | | |
|---|---|---|---|---|---|---|---|---|
| | Ex. 1 | Ex. 2 | Ex. 5 | Ex. 6 | Ex. 7 | Ex. 8 | Ex. 9 | Ex. 11 |
| *S. aureus* 209p JC-1 | 0.78 | 0.39 | 1.56 | 0.78 | 0.39 | 0.39 | 0.1 | 0.1 |
| *S. aureus* Smith | 1.56 | 0.78 | 3.13 | 1.56 | 0.78 | 0.39 | 0.2 | 0.1 |
| *S. aureus* 167 | 12.5 | 6.25 | 50 | 12.5 | 12.5 | 12.5 | 6.25 | 6.25 |
| *S. aureus* 195 | 12.5 | 3.13 | 50 | 6.25 | 6.25 | 3.13 | 1.56 | 1.56 |
| *S. epidemidis* IAM 1296 | 0.39 | 0.2 | 0.78 | 0.39 | 0.2 | 0.1 | 0.05 | 0.05 |
| *M. luteus* ATCC 9341 | 0.2 | 0.05 | 0.39 | 0.1 | 0.2 | 0.05 | 0.05 | 0.05 |
| *B. subtilis* ATCC 6633 | 0.39 | 0.39 | 0.78 | 0.39 | 0.78 | 0.78 | 0.2 | 0.1 |
| *E. coli* NHIJ JC-2 | 1.56 | 0.78 | 6.25 | 0.78 | 0.78 | 0.39 | 0.2 | 0.1 |
| *E. coli* CSH2(RK1) | 3.13 | 1.56 | 12.5 | 1.56 | 1.56 | 1.56 | 0.39 | 0.39 |
| *E. coli* CSH2(RE45) | 3.13 | 0.78 | 6.25 | 0.78 | 1.56 | 0.39 | 0.39 | 0.2 |
| *K. pneuminiae* IFO 3317 | 0.78 | 0.39 | 3.13 | 0.39 | 0.78 | 0.2 | 0.2 | 0.1 |
| *K. pneuminiae* No. 42 | 1.56 | 0.78 | 6.25 | 1.56 | 1.56 | 0.78 | 0.39 | 0.39 |
| *P. mirabilis* IFO 3849 | 1.56 | 1.56 | 3.13 | 0.78 | 0.78 | 0.2 | 0.39 | 0.2 |
| *P. vulugaris* OX-19 | 3.13 | 1.56 | 6.25 | 1.56 | 1.56 | 0.2 | 0.39 | 0.1 |
| *M. morganii* IFO 3848 | 1.56 | 0.78 | 3.13 | 0.39 | 0.39 | 0.05 | 0.1 | 0.05 |
| *S. marcescens* IAM 1184 | 0.78 | 0.39 | 6.25 | 0.78 | 0.78 | 0.39 | 0.39 | 0.39 |
| *S. marcescens* No. 16-2 | >100 | 100 | >100 | >100 | >100 | 100 | 50 | 100 |
| *E. cloacae* ATCC 13407 | 3.13 | 6.25 | 25 | 6.25 | 6.25 | 3.13 | 1.56 | 6.25 |
| *E. cloacae* Nek39 | 100 | 100 | 100 | 50 | 100 | 50 | 50 | >100 |
| *C. freundii* ATCC 8090 | 1.56 | 0.78 | 6.25 | 0.78 | 0.78 | 0.2 | 0.2 | 0.2 |
| *P. aeruginosa* NCTC 10490 | 0.78 | 0.78 | 3.13 | 0.78 | 6.25 | 1.56 | 3.13 | 3.13 |
| *P. aeruginosa* AKR17 | >100 | >100 | >100 | >100 | >100 | >100 | >100 | >100 |
| *P. cepacia* | >100 | >100 | >100 | >100 | >100 | >100 | >100 | >100 |
| *A. faecalis* ATCC 3750 | 6.25 | 3.13 | 12.5 | 3.13 | 3.13 | 0.78 | 3.13 | 1.56 |
| *A. calcoaceticos* T59 | 3.13 | 1.56 | 12.5 | 6.25 | 25 | 12.5 | 12.5 | 25 |

Table 1 (continued)

| Strains tested | Drugs tested | | | | |
|---|---|---|---|---|---|
| | Ex. 12 | Ex. 13 | Ex. 14 | FMOX | CTM |
| *S. aureus* 209p JC-1 | 0.2 | 0.2 | 0.2 | 0.39 | 1.56 |
| *S. aureus* Smith | 0.39 | 0.39 | 0.39 | 0.39 | 1.56 |
| *S. aureus* 167 | 6.25 | 6.25 | 12.5 | 50 | >100 |
| *S. aureus* 195 | 1.56 | 3.13 | 6.25 | 3.13 | >100 |
| *S. epidemidis* IAM 1296 | 0.05 | 0.1 | 0.1 | 0.2 | 0.39 |
| *M. luteus* ATCC 9341 | 0.025 | 0.025 | 0.05 | 0.1 | 0.39 |
| *B. subtilis* ATCC 6633 | 0.2 | 0.2 | 0.2 | 0.39 | 0.39 |
| *E. coli* NHIJ JC-2 | 0.39 | 0.2 | 0.39 | 0.1 | 0.2 |
| *E. coli* CSH2(RK1) | 0.78 | 0.39 | 0.78 | 0.1 | 0.2 |
| *E. coli* CSH2(RE45) | 0.39 | 0.39 | 0.39 | 0.1 | 0.2 |
| *K. pneuminiae* IFO 3317 | 0.2 | 0.2 | 0.39 | 0.05 | 0.2 |
| *K. pneuminiae* No. 42 | 0.78 | 0.78 | 0.78 | 0.1 | 0.39 |
| *P. mirabilis* IFO 3849 | 0.39 | 0.39 | 0.39 | 0.39 | 0.78 |
| *P. vulugaris* OX-19 | 0.39 | 0.2 | 0.39 | 0.39 | 0.39 |
| *M. morganii* IFO 3848 | 0.1 | 0.1 | 0.2 | 0.39 | 0.2 |
| *S. marcescens* IAM 1184 | 0.78 | 0.39 | 0.78 | 0.2 | 6.25 |
| *S. marcescens* No. 16-2 | 100 | 100 | >100 | 100 | >100 |
| *E. cloacae* ATCC 13407 | 3.13 | 3.13 | 3.13 | 50 | 50 |
| *E. cloacae* Nek39 | 100 | >100 | 100 | >100 | >100 |
| *C. freundii* ATCC 8090 | 0.39 | 0.2 | 0.2 | 0.39 | 0.78 |
| *P. aeruginosa* NCTC 10490 | 6.25 | 1.56 | 3.13 | >100 | >100 |
| *P. aeruginosa* AKR17 | >100 | >100 | >100 | >100 | >100 |
| *P. cepacia* | >100 | >100 | >100 | 50 | >100 |
| *A. faecalis* ATCC 3750 | 1.56 | 1.56 | 3.13 | 0.1 | 6.25 |
| *A. calcoaceticos* T59 | 12.5 | 12.5 | 12.5 | >100 | >100 |

Table 2: MIC (μg/ml) on standard strains

| Strains tested | Drugs tested | | | | |
|---|---|---|---|---|---|
| | Ex. 15 | Ex. 16 | Ex. 17 | Ex. 18 | Ex. 19 |
| *S. aureus* 209p JC-1 | 0.39 | 0.2 | 0.39 | 0.78 | 0.2 |
| *S. aureus* Smith | 0.39 | 0.39 | 0.78 | 1.56 | 0.39 |
| *S. aureus* 167 | 6.25 | 3.13 | 6.25 | 25 | 6.25 |
| *S. aureus* 195 | 6.25 | 1.56 | 3.13 | 12.5 | 1.56 |
| *S. epidemidis* IAM 1296 | 0.1 | 0.1 | 0.2 | 0.39 | 0.1 |
| *M. luteus* ATCC 9341 | 0.05 | 0.025 | 0.1 | 0.2 | 0.1 |
| *B. subtilis* ATCC 6633 | 0.39 | 0.2 | 0.39 | 0.78 | 0.39 |
| *E. coli* NHIJ JC-2 | 0.2 | 0.1 | 0.39 | 0.2 | 0.39 |
| *E. coli* CSH2(RK1) | 0.78 | 0.78 | 0.78 | 6.25 | 0.78 |
| *E. coli* CSH2(RE45) | 0.39 | 0.2 | 0.78 | 0.39 | 0.39 |
| *K. pneuminiae* IFO 3317 | 0.2 | 0.1 | 0.39 | 0.2 | 0.2 |
| *K. pneuminiae* No. 42 | 0.39 | 0.39 | 0.78 | 1.56 | 0.78 |
| *P. mirabilis* IFO 3849 | 0.2 | 0.2 | 0.39 | 0.2 | 0.39 |
| *P. vulugaris* OX-19 | 0.2 | 0.1 | 0.39 | 0.2 | 0.39 |
| *M. morganii* IFO 3848 | 0.1 | 0.05 | 0.2 | 0.05 | 0.2 |
| *S. marcescens* IAM 1184 | 0.2 | 0.2 | 0.39 | 0.39 | 0.2 |
| *S. marcescens* No. 16-2 | 100 | >100 | >100 | >100 | 100 |
| *E. cloacae* ATCC 13407 | 6.25 | 12.5 | 6.25 | 50 | 3.13 |
| *E. cloacae* Nek39 | 50 | >100 | 50 | >100 | 50 |
| *C. freundii* ATCC 8090 | 0.2 | 0.2 | 0.39 | 0.39 | 0.39 |
| *P. aeruginosa* NCTC 10490 | 1.56 | 1.56 | 3.13 | 12.5 | 3.13 |
| *P. aeruginosa* AKR17 | >100 | >100 | >100 | >100 | >100 |
| *P. cepacia* | >100 | >100 | >100 | >100 | >100 |
| *A. faecalis* ATCC 3750 | 1.56 | 1.56 | 3.13 | 1.56 | 3.18 |
| *A. calcoaceticos* T59 | 12.5 | 12.5 | 12.5 | 25 | 6.25 |

Table 2 (continued)

| Strains tested | Drugs tested | | | | |
|---|---|---|---|---|---|
| | Ex. 20 | Ex. 21 | Ex. 22 | FMOX | CTM |
| *S. aureus* 209p JC-1 | 0.2 | 0.2 | 0.2 | 0.39 | 0.78 |
| *S. aureus* Smith | 0.39 | 0.39 | 0.2 | 0.39 | 0.78 |
| *S. aureus* 167 | 6.25 | 3.13 | 1.56 | 50 | >100 |
| *S. aureus* 195 | 3.31 | 1.56 | 0.78 | 3.13 | >100 |
| *S. epidemidis* IAM 1296 | 0.05 | 0.1 | 0.1 | 0.2 | 0.39 |
| *M. luteus* ATCC 9341 | 0.025 | 0.025 | 0.025 | 0.1 | 0.39 |
| *B. subtilis* ATCC 6633 | 0.2 | 0.2 | 0.2 | 0.2 | 0.39 |
| *E. coli* NHIJ JC-2 | 0.2 | 0.1 | 0.1 | 0.1 | 0.2 |
| *E. coli* CSH2(RK1) | 0.39 | 0.2 | 0.2 | 0.1 | 0.2 |
| *E. coli* CSH2(RE45) | 0.2 | 0.1 | 0.1 | 0.1 | 0.39 |
| *K. pneuminiae* IFO 3317 | 0.1 | 0.1 | 0.05 | 0.05 | 0.2 |
| *K. pneuminiae* No. 42 | 0.39 | 0.2 | 0.1 | 0.1 | 0.39 |
| *P. mirabilis* IFO 3849 | 0.2 | 0.2 | 0.1 | 0.2 | 0.39 |
| *P. vulugaris* OX-19 | 0.2 | 0.1 | 0.1 | 0.39 | 0.39 |
| *M. morganii* IFO 3848 | 0.1 | 0.05 | 0.025 | 0.39 | 0.2 |
| *S. marcescens* IAM 1184 | 0.1 | 0.1 | 0.1 | 0.2 | 3.13 |
| *S. marcescens* No. 16-2 | 100 | 50 | 25 | 100 | >100 |
| *E. cloacae* ATCC 13407 | 12.5 | 1.56 | 0.78 | 100 | >100 |
| *E. cloacae* Nek39 | 50 | 50 | 25 | 100 | >100 |
| *C. freundii* ATCC 8090 | 0.2 | 0.1 | 0.1 | 0.2 | 0.78 |
| *P. aeruginosa* NCTC 10490 | 0.78 | 6.25 | 3.13 | >100 | 100 |
| *P. aeruginosa* AKR17 | >100 | >100 | >100 | >100 | >100 |
| *P. cepacia* | >100 | >100 | >100 | 100 | >100 |
| *A. faecalis* ATCC 3750 | 0.78 | 0.78 | 1.56 | 0.1 | 3.13 |
| *A. calcoaceticos* T59 | 6.25 | 12.5 | 12.5 | 100 | >100 |

Efect of the Invention:

Novel cephem compounds having a strong and broad antimicrobial spectrum, especially an excellent antimicrobial activity on methicillin-resistant *Staphylococcus aureus*, are provided by the present invention.

## Claims

1. A cephem compound represented by the following general formula (I) :

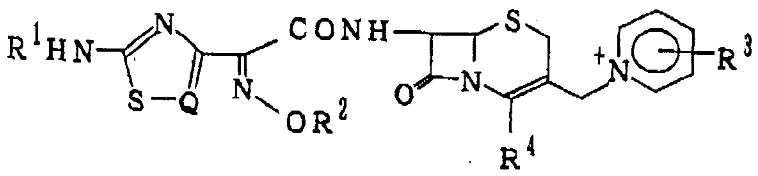

(I)

wherein $R^1$ represents a hydrogen atom or an amino group-protecting group; $R^2$ represents a hydrogen atom or a hydroxyimino group-protecting group; $R^3$ is a 3- or 4-position substituent group on the pyridinium ring and represents a carbamoylmethyl group, a ureido group, a thiazol group which may be substituted with an amino group, a thiadiazol group which may be substituted with an amino group, an oxadiazol group which may be substituted with an amino group, or a group represented by the following general formula (1)

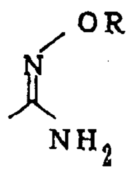

(1)

(1)

(wherein R represents a hydrogen atom or an alkyl group); $R^4$ represents a carboxyl or carboxylate group which may be protected; and Q represents CH or N; and a physiologically or pharmaceutically acceptable salt thereof.

2. An antimicrobial or antibacterial agent which contains as an active ingredient at least one of the cephem compounds represented by the general formula (I) described in claim 1 and/or a physiologically or pharmaceutically acceptable salt thereof.

3. A compound according to claim 1 for pharmaceutical use.

4. Use of a compound according to claim 1 in the manufacture of an antimicrobial.

5. Use according to claim 4 wherein the antimicrobial is an antibacterial.

European Patent Office

# EUROPEAN SEARCH REPORT

Application Number
EP 94 30 5665

| Category | Citation of document with indication, where appropriate, of relevant passages | Relevant to claim | CLASSIFICATION OF THE APPLICATION (Int.Cl.6) |
|---|---|---|---|
| P,X | EP-A-0 554 004 (KATAYAMA SEIYAKUSYO CO. LTD.)<br>* claims 1,3-9; examples 4,5,7,9 *<br>--- | 1-5 | C07D501/46<br>A61K31/545 |
| Y | EP-A-0 359 291 (MOCHIDA PHARMACEUTICAL CO. LTD.)<br>* page 13, table 1; pages 34-38, examples 15 and 16 *<br>--- | 1-5 | |
| Y | EP-A-0 047 977 (CIBA-GEIGY AG)<br>* page 1, formulae (I) and (A); page 6, lines 1-11; pages 67, 68, Example 14; page 69, Example 18; page 73, second, third, and fourth compound; claims 1,18-21 *<br>--- | 1-5 | |
| A | EP-A-0 027 599 (FUJISAWA PHARMACEUTICAL CO. LTD.)<br>* claims 1,18 *<br>--- | 1-5 | |
| A | EP-A-0 042 154 (FUJISAWA PHARMACEUTICAL CO. LTD.)<br>* claims 1,17 *<br>--- | 1-5 | TECHNICAL FIELDS SEARCHED (Int.Cl.6) |
| A | EP-A-0 064 740 (HOECHST AG)<br>* claims 1,7 *<br>--- | 1-5 | C07D |
| A | JOURNAL OF ANTIBIOTICS, vol.XXXVII, no.5, 1984<br>pages 557 - 571<br>J. GOTO ET AL. 'Studies of 7-beta-[2-(Aminoaryl)acetamido]-cephalosporin derivatives. III. Synthesis and structure-activity relationships in the aminothiadiazole series'<br>* page 561, table 3 *<br>---<br>-/-- | 1-5 | |

The present search report has been drawn up for all claims

| Place of search | Date of completion of the search | Examiner |
|---|---|---|
| BERLIN | 24 November 1994 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 30 5665

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | PATENT ABSTRACTS OF JAPAN vol. 14, no. 439 (C-0761) 19 September 1990 & JP-A-02 172 989 (DAI ICHI SEIYAKU CO LTD) 4 July 1990 ----- | 1 | |
| | | | TECHNICAL FIELDS SEARCHED (Int.Cl.6) |

The present search report has been drawn up for all claims

| Place of search | Date of completion of the search | Examiner |
|---|---|---|
| BERLIN | 24 November 1994 | Hass, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)